(19) Europäisches Patentamt / European Patent Office / Office européen des brevets

(11) **EP 3 068 223 B1**

(12) **EUROPEAN PATENT SPECIFICATION**


(45) Date of publication and mention of the grant of the patent: **01.01.2020 Bulletin 2020/01**

(21) Application number: **14862229.3**

(22) Date of filing: **23.06.2014**

(51) Int Cl.:
***A01N 43/04*** (2006.01) ***A61K 9/06*** (2006.01)
***A61K 47/36*** (2006.01) ***A61K 31/57*** (2006.01)

(86) International application number: **PCT/US2014/043649**

(87) International publication number: **WO 2015/073066 (21.05.2015 Gazette 2015/20)**


(54) **GLYCOL CHITIN BASED THERMOSENSITIVE HYDROGEL FOR VAGINAL DELIVERY OF PROGESTERONE**

GLYCOLCHITINBASIERTES WÄRMEEMPFINDLICHES HYDROGEL ZUR VAGINALEN VERABREICHUNG VON PROGESTERON

HYDROGEL THERMOSENSIBLE À BASE DE GLYCOL-CHITINE POUR ADMINISTRATION DE PROGESTÉRONE PAR VOIE VAGINALE


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2013 US 201361962609 P**

(43) Date of publication of application: **21.09.2016 Bulletin 2016/38**



(73) Proprietors:
- **University of Utah Research Foundation Salt Lake City, UT 84108 (US)**
- **Janat-Amsbury, Margit, Maria Salt Lake City, UT 84104 (US)**
- **THE INDUSTRY & ACADEMIC COOPERATION IN CHUNGNAM NATIONAL UNIVERSITY (IAC) Daejeon 34134 (KR)**
- **Peterson, Charles, Matthew Salt Lake City, UT 84112 (US)**

(72) Inventors:
- **JANAT-AMSBURY, Margit, Maria Salt Lake City, UT 84104 (US)**
- **HUH, Kang, Moo Yuseong-gu Daejeon (US)**
- **PETERSON, Charles, Matthew Salt Lake City, UT 84112 (US)**

(74) Representative: **Grünecker Patent- und Rechtsanwälte PartG mbB Leopoldstraße 4 80802 München (DE)**



(56) References cited:

| | |
|---|---|
| **WO-A1-2008/154240** | **WO-A1-2008/154240** |
| **US-A- 4 038 389** | **US-A- 5 362 720** |
| **US-A- 5 543 450** | **US-A1- 2002 012 703** |
| **US-A1- 2003 211 974** | **US-A1- 2004 132 690** |
| **US-A1- 2005 085 453** | **US-A1- 2006 115 532** |
| **US-A1- 2008 268 063** | **US-B2- 8 445 465** |

- **MARTIN ET AL.: 'Sustained Buccal delivery of the hydrophobic drug using physically cross linked palmytol glycol chitosan hydrogels;' EUR. J. PHAR. BIOPHARM. vol. 55, 2003, pages 35 - 45, XP004404956**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).





Printed by Jouve, 75001 PARIS (FR)

## Description

Progesterone is a steroidal hormone that participates in various aspects of female reproductive physiology, such as endometrial proliferation during the menstrual cycle, ovulation, implantation of fertilized oocytes, and maintenance of pregnancy. Other physiological actions of progesterone that do not involve the reproductive system include proliferation of normal breast tissue, prevention of bone loss, and regulation of sexual responsive signals in the brain. Therapeutic uses for progesterone include the management of several female reproductive conditions, such as the prevention of preterm birth, luteal phase support, and in hormonal replacement therapy. Furthermore, progesterone has been used as part of fertility-sparing treatments for endometrial hyperplasia and primary endometrial cancer due in part to its antiestrogenic effects on endometrial tissue.

Therapeutic doses of progesterone are most often provided via oral, intramuscular, and vaginal routes of administration. Oral administration is convenient but results in relatively poor bioavailability, reaching only low endometrial concentrations due to the hepatic first pass effect and high affinity to plasma proteins. Synthetic progesterone (progestins) may offer improved oral bioavailability but are commonly associated with undesirable side effects ranging from acne and water retention to depression. Intramuscular injections offer an alternative administration route that facilitates cellular absorption. But pain, abscesses/infections, or in some cases neuropathies associated with such injections can reduce patient compliance.

WO2008154240 (A1) discloses pharmaceutical compositions appropriate for the vaginal administration of steroids, to be used in a hormone replacement therapy, during menopause and to support the luteal phase of in vitro fertilization cycles. Compositions are characterized by the incorporation of polymers and disintegrators combinations, that allow an adequate mucous adherence and bio-availability of steroid hormones in the endometrium. This administration method reduces the systemic levels of steroids, allows a longer term in between doses, thus reducing side effects, and it prevents residues gathering inside the vaginal cavity.

US 8 445 465 (B2) discloses a glycol chitosane derivative, a preparation method thereof and a drug delivery system comprising the same. More specifically, US 8 445 465 (B2) discloses a glycol chitosan derivative, which can form nano-sized self-assembled structures and has both temperature sensitivity and biodegradability so as to be suitable for use as a drug delivery system, as well as a preparation method thereof and a drug delivery system comprising the same.

The present disclosure provides a novel alternative for convenient, safe, and effective progesterone delivery via the vaginal route.

Specifically, the present invention provides a composition for intravaginal delivery according to claim 1, the composition comprising:

glycol chitin having a degree of acetylation of at least 80%; and
progesterone;
the composition has a pH from 3.5 to 5.5, and exhibits a gelation temperature from 30°C to 36°C.

In another embodiment, the present invention provides a composition for use in a method for the sustained intravaginal delivery of progesterone, the method comprising intravaginally administering to a subject in need thereof said composition comprising:

glycol chitin having a degree of acetylation of at least 80%; and
progesterone;
wherein the composition has a pH from 3.5 to 5.5, and exhibits a gelation temperature from 30°C to 36°C.

In another embodiment, the present invention provides a kit for the intravaginal delivery of progesterone, comprising:

a. the composition of claim 1, and
b. an application device.

Preferred embodiments are set forth in the subclaims.

FIG. 1 is a chart showing the observed gelation temperatures for compositions comprising differing wt% of glycol chitin with a 90% degree of acetylation at pH 4.2.

FIG. 2 is a chart showing the results of a rheological analysis demonstrating mucoadhesion between glycol chitin and mucin.

FIG. 3 is a series of charts showing the effects of the degree of acetylation, glycol chitin concentration, and salt concentration on gelation temperatures of glycol chitin compositions.

FIG. 4 is two charts showing elastic modulus (G') changes exhibited by glycol chitin hydrogel with thermal heating and cooling cycles.

FIG. 5 is a chart showing gelation temperatures measured for compositions comprising progesterone and glycol chitin at pH 4.2 with varying concentrations of glycol chitin.

FIG. 6 is a chart showing gelation temperatures measured for compositions comprising progesterone and glycol chitin with varying pH.

FIG. 7 is a series of charts showing temperature sweep rheometer measurements of G' (elastic modulus) and G" (viscous modulus) used to determine gelation temperatures for compositions comprising progesterone and glycol chitin (GC) having 80% degree of acetylation (DA).

FIG. 8 is a chart showing gelation temperatures recorded for compositions containing glycol chitin with and without 1.0 mg/ml (0.1%) progesterone (P4).

FIG. 9 is a chart showing rapid gelation of a composition comprising progesterone and glycol chitin within seconds after exposure to 37°C temperature, as indicated by rheological measurements of G' (elastic modulus) and G" (viscous modulus).

FIG. 10 is a series of charts showing mechanical characteristics of gels formed by compositions comprising progesterone and glycol chitin (GC-P4).

FIG. 11 is a series of charts showing viscosity and gel strength measurements of gels formed by compositions comprising progesterone and glycol chitin (GC-P4) when exposed to shear forces with or without dilution with vaginal fluid simulant (VFS) or seminal fluid simulant (SFS).

FIG. 12 is a series of charts showing in vitro release and bioactivity of progesterone (P4) from gels formed from progesterone-glycol chitin compositions (GC-P4).

FIG. 13 is two charts showing in vitro measurements of lysozyme biodegradation of gels formed from compositions comprising glycol chitosan and glycol chitins of varying degrees of acetylation (DA) as well as cell viability measurements performed on cells exposed to such compositions.

FIG. 14 is a series of charts showing in vitro measurements of biocompatibility of compositions comprising glycol chitin (GC) with and without progesterone (P4), as well as in comparison to other commercial compositions.

FIG. 15 is a diagram showing possible intramolecular and intermolecular interactions in a composition comprising glycol chitin and progesterone

FIG. 16 is a pair of tables showing the composition of vaginal fluid simulant (VFS) and seminal fluid simulant (SFS).

Described herein are pharmaceutical compositions, methods, and kits for vaginal delivery of progesterone. The disclosure describes the use of glycol chitin in preparing a new thermosensitive progesterone hydrogel for vaginal delivery, providing a better alternative to currently available vaginal progesterone formulations. Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

### 1. Definitions

The terminology used herein is for the purpose of describing particular embodiments only and is not intended to be limiting. As used in the specification and the appended claims, the singular forms "a" and "the" include plural references unless the context clearly dictates otherwise.

For the recitation of numeric ranges in this disclosure, each intervening number with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

As used herein, the term "about" is used synonymously with the term "approximately." Accordingly, the use of the term "about" includes values slightly outside the cited values, namely, plus or minus 10%. Such values are thus encompassed by the scope of the claims reciting the terms "about" and "approximately." For example, the term "about" as used in connection with the size of a polymeric molecule is intended to account for the fact that a sample of such molecules generally has a distribution of sizes and molecular weights of approximately plus or minus 10%. Similarly, compositions formed using polymeric molecules also will have a distribution of sizes and molecular weights.

The terms "administer," "administering," "administered," or "administration" refer to any manner of providing a compound or a pharmaceutical composition to a subject or patient. Unless specified, administration using a particular route can be accomplished through any means known by those skilled in the art.

"Effective amount," as used herein, refers to a dosage of a compound or a composition effective for eliciting a desired effect. The term may also refer to an amount effective at bringing about a desired in vivo effect in an animal or human.

As used herein, the term "subject" is intended to include human and non-human animals. A human subject may be, for example, a healthy human or a human patient having a disorder such as endometrial cancer. The term "non-human animals" includes all vertebrates, e.g. non-mammals (such as chickens, amphibians, reptiles), and mammals, such as non-human primates, domesticated and/or agriculturally useful animals (such as sheep, dogs, cats, cows, pigs, etc.), and rodents (such as mice, rats, hamsters, guinea pigs, etc.).

As used herein, the term "treat" or "treating" a subject having a disorder refers to administering a compdund or a composition to the subject, such that at least one symptom of the disorder is cured, healed, alleviated, relieved, altered, remedied, ameliorated, or improved. Treating includes administering an amount effective to alleviate, relieve, alter, remedy, ameliorate, cure, improve, or affect the disorder or the symptoms of the disorder. Treating a disorder may inhibit deterioration or worsening of a symptom of a disorder.

"Chitosan" refers to a cationic polysaccharide derived from chitin, a biopolymer found in the shells of crustaceans. Generally, chitosan is obtained by removing about 50% or more of acetyl groups of C2 acetamide from chitin, and chitosan generally has a degree of acetylation of less than 50%. Chitosan comprises β(1,4)-linked N-acetyl-D-glucosamine and D-glucosamine units. Chitosan exhibits relatively poor water solubility.

"Glycol chitosan" is a chitosan derivative that exhibits improved water solubility compared to chitosan due to the introduction of hydrophilic ethylene glycol groups. Glycol chitosan generally has a degree of acetylation of less than 50%. An exemplary structure representing a glycol chitosan is as follows, where *n* is an integer ranging from about 10 to about 10,000:

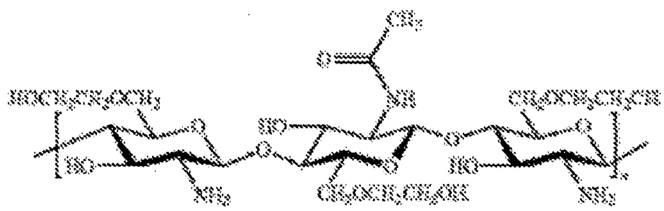

"Glycol chitin" refers to an N-acetylated derivative of glycol chitosan having a degree of acetylation of at least 50%. An exemplary structure representing a fully N-acetylated glycol chitin is as follows, where *n* is an integer ranging from about 10 to about 10,000:

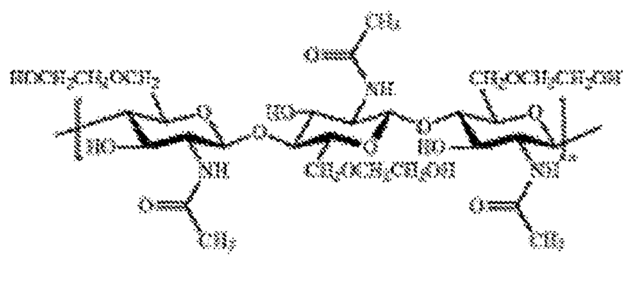

Glycol chitin need not be fully N-acetylated, and in addition to the exemplary structure shown above, other examples of

glycol chitin may retain some proportion of non-acetylated primary amine groups along the polymer chain. Thus, glycol chitins may exhibit varying degrees of acetylation, ranging from about 50% to about 100%.

"Degree of acetylation" refers to the ratio or percentage of amine groups along the backbone of a chitosan or chitosan derivative molecule (such as glycol chitosan or glycol chitin) that are acetylated. Expressions of a degree of acetylation may represent an average for a population of polymeric molecules, and some variation may exist in the exact degree of acetylation between individual molecules in the population. Thus, in some embodiments, the degree of acetylation for a given sample may be expressed as falling within an enumerated range, such as, for example, 90% ± 2.0%.

"Degree of polymerization" refers to the average number of sugar monomers per individual polymeric molecule in a sample comprising chitosan or a chitosan derivative, such as glycol chitin.

"Gelation temperature" means the temperature at which a glycol chitin preparation undergoes a sol-gel transition under a given set of conditions (e.g., pH, polymer concentration etc.).

## 2. Compositions

Effective vaginal delivery of progesterone presents many challenges, and vaginal progesterone formulations such as suppositories and creams often perform poorly. The vaginal environment-including body temperature (37°C), acidic vaginal pH (between about pH 3.5 and about pH 5.5, most often between about pH 3.8 and pH 4.5), vaginal fluids, abundant mucin, and self-cleansing mechanisms-affects vaginal residence times of therapeutic formulations. Moreover, most creams leak out of the vaginal cavity and can often result in hygiene issues, vaginal irritation, and frequent yeast infections.

Thermosensitive polymers possessing a reversible sol-gel transition behavior in aqueous media, such as copolymers of poly(ethylene oxide), poly(propylene oxide), and copolymers of N-isopropylacrylamide, have been used for vaginal drug delivery. Such materials may offer improvements over traditional creams and suppositories, especially if combined with mucoadhesive agents, but their clinical applications have been limited by their lack of biodegradability, biocompatibility, and physical stability.

Another thermo-reversible sol-gel transition polymer, glycol chitin, can be synthesized by N-acetylation of glycol chitosan. In contrast to typical sol-gel transition polymers structurally based on synthetic block copolymer structures, glycol chitin is an amphiphilic polymer with good biocompatibility and biodegradability. The introduction of hydrophobic acetyl groups by N-acetylation renders glycol chitin amphiphilic, and glycol chitin has improved solubility in organic solvents. In addition, glycol chitin can form self-assembled nanoparticles in an aqueous media by a hydrophobic interaction between hydrophobic moieties in order to reduce the surface free energy. The hydrophilic shell can act as a barrier against interactions with cells, proteins, and biological tissues, and the hydrophobic core can act as a space for storing various hydrophobic agents. Glycol chitin exhibits a sol-gel phase transition behavior according to a change in temperature and is one of few polysaccharide-based polymers autonomously showing a negatively thermosensitive sol-gel transition around body temperature without involvement of any crosslinking, grafting, or blending systems. Also, glycol chitin is readily biodegradable at least in part because its acetyl groups are sensitive to digestive enzymes such as lysozyme.

The disclosed compositions comprise glycol chitin and progesterone. Glycol chitin may be produced, for example, using methods described in U.S. Patent 8,445,465,. In addition, in some embodiments, the compositions may include synthetic progesterone analogs (progestins) known to those of ordinary skill in the art (such as, for example, medroxyprogesterone, norethindrone, or megestrol) instead of, or in addition to, progesterone.

The inventors have determined that the properties of the disclosed compositions, such as gelation temperature, vary according to several parameters, including pH, glycol chitin concentration, progesterone concentration, the degree of acetylation of glycol chitin, and the degree of polymerization of glycol chitin. The disclosed compositions exhibit thermoreversible sol-gel transition properties with fast gelation kinetics, in particular, the compositions are freely flowable at room temperature but quickly transition to a durable gel under typical physiological conditions in the vaginal environment (i.e., at body temperature and vaginal pH).

In some embodiments, the disclosed compositions may include glycol chitin having a degree of acetylation of preferably between 85% and 95%. Thus, the compositions may include glycol chitin having a degree of acetylation of, for example, about 85%, about 86%, about 87%, about 88%, about 89%, about 90%, about 91%, about 92%, about 93%, about 94%, or about 95%. In some embodiments, the compositions may include glycol chitin having a degree of acetylation of, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, or at least 95%. In some embodiments, the compositions may include glycol chitin having a degree of acetylation of at most 99%, at most 95%, at most 90%, at most 85%, In some embodiments, the degree of acetylation may be determined as an average of replicate measurements taken to determine the average degree of acetylation for the polymer molecules in a sample. In some embodiments, the degree of acetylation for a sample may be expressed as falling with a range determined via replicate measurements, for example, as a given degree of acetylation ± 2.0%.

The disclosed compositions may be provided as aqueous preparations, though other solvents or co-solvents known to those of skill in the art, such as, for example, alcohols, may also be used in some embodiments within the scope of the invention. In addition, in some embodiments, the disclosed compositions may include buffered solutions known to those of skill in the art and formulated to have a desired pH at suitable concentrations. Suitable buffers may include, for example, such as, for example, phosphate buffered saline, citrate buffer, Tris buffer, citrate-phosphate buffer, sodium acetate buffer, or glycine-HCl buffers.

The pH of the disclosed compositions is preferably between 4.0 and 4.4. Thus, the pH of the compositions may be, for example, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5. In some embodiments, the pH of the composition may be determined or influenced by a buffer included in the composition.

In some embodiments, the disclosed compositions may include glycol chitin in an amount of between about 2% and about 10% by weight, preferably between about 3% and about 8% by weight, and more preferably between about 4% and about 6% by weight. The compositions may include, by weight, about 2% glycol chitin, about 3% glycol chitin, about 4% glycol chitin, about 5% glycol chitin, about 6% glycol chitin, about 7% glycol chitin, about 8% glycol chitin, about 9% glycol chitin, or about 10% glycol chitin.

In addition, in some embodiments the glycol chitin included in the disclosed compositions may have an average degree of polymerization of between about 100 and about 10,000, preferably between about 200 and about 1,000, and more preferably between about 400 and about 800. In some embodiments, the average degree of polymerization of the glycol chitin may be at least about 100, at least about 200, at least about 300, at least about 400, at least about 500, at least about 600, at least about 700, at least about 800, at least about 900, or at least about 1,000. In some embodiments, the compositions may include glycol chitin having an average degree of polymerization of at most about 10,000, at most about 5,000, at most about 1,000, at most about 900, at most about 800, at most about 700, at most about 600, at most about 500, at most about 400, at most about 300, at most about 200, or at most about 100.

In some embodiments, the disclosed compositions may include progesterone, for example, in an amount between about 0.1 mg/ml and about 2.0 mg/ml, in an amount between about 0.2 mg/ml and about 1.5 mg/ml, or in an amount between about 0.6 mg/ml and about 1.2 mg/ml. Thus, for example, in some embodiments, the disclosed compositions may include about 0.5 mg/ml progesterone, about 0.6 mg/ml progesterone, about 0.7 mg/ml progesterone, about 0.8 mg/ml progesterone, about 0.9 mg/ml progesterone, about 1.0 mg/ml progesterone, about 1.1 mg/ml progesterone, about 1.2 mg/ml progesterone, about 1.3 mg/ml progesterone, about 1.4 mg/ml progesterone, about 1.5 mg/ml progesterone, about 1.6 mg/ml progesterone, about 1.7 mg/ml progesterone, about 1.8 mg/ml progesterone, about 1.9 mg/ml progesterone, or about 2.0 mg/ml progesterone. In some embodiments, the compositions may include at most about 2.0 mg/ml progesterone, at most about 1.9 mg/ml progesterone, at most about 1.8 mg/ml progesterone, at most about 1.7 mg/ml progesterone, at most about 1.6 mg/ml progesterone, at most about 1.5 mg/ml progesterone, at most about 1.4 mg/ml progesterone, at most about 1.3 mg/ml progesterone, at most about 1.2 mg/ml progesterone, at most about 1.1 mg/ml progesterone, at most about 1.0 mg/ml progesterone, at most about 0.9 mg/ml progesterone, or at most about 0.8 mg/ml progesterone.

The compositions achieve physical and chemical properties that allow for rapid gel formation upon vaginal administration. Thus, for example, in some embodiments, the disclosed compositions have a gelation temperature of 30°C to 36°C under acidic conditions between pH 3.5 and pH 5.5. In some embodiments, the disclosed compositions may exhibit a gelation temperature of about 30°C, about 31°C, about 32°C, about 33°C, about 34°C, about 35°C, or about 36°C at vaginal pH. Thus, for example, some disclosed compositions might have a gelation temperature of 35°C at pH 3.8, some disclosed compositions might have a gelation temperature of 34°C at pH 4.2, and some disclosed compositions might have a gelation temperature of 33°C at pH 4.8. The gelation temperature of each particular composition at each particular pH within the scope of the invention will be influenced by the makeup of the composition, including the amount of glycol chitin, the degree of acetylation of the glycol chitin, and the amount of progesterone, among other factors. Further illustration of disclosed compositions can be found in the Examples.

The disclosed compositions offer various advantages for vaginal progesterone administration such as avoidance of the hepatic first pass effect, ease of use, and the provision of site-specific treatment for various gynecological disorders. In addition, the vaginal administration of progesterone using the disclosed compositions shows a preferential absorption and distribution to the uterus based on the uterine first pass effect, a direct transport mechanism for drugs between vagina and uterus. The disclosed compositions also provide advantageous mucoadhesive and mechanical properties, facilitating longer resident times of the disclosed compositions in the vagina without breakdown or leakage. The gels formed by the disclosed glycol-chitin/progesterone compositions are stable in the vaginal environment, safe for vaginal tissues, and effective for sustained, steady progesterone delivery. Leakage of vaginal formulations often causes vaginal discharge accompanied by itching and leads to vulvar tissue irritation, resulting in patient discomfort and reduced user acceptance. The leakage reduction provided with the disclosed compositions provides more accurate dosing and improved patient compliance, which may reduce or eliminate the need for frequent applications.

In addition, the disclosed compositions exhibit favorable rheological characteristics such as stability and spreadability in vaginal like environment. The gel microstructure of the disclosed compositions is more resistant to external stress compared to Crinone®, a commercially available progesterone gel. Accordingly, the disclosed gels maintain a stable gel under physiological conditions and are less prone to liquefy. In addition, the preferred shear thinning non-Newtonian behavior that the disclosed gels retain under increasing shear indicates a satisfactory distribution across vaginal walls. Besides shearing, there are several other factors in the vagina that can control a gel flow behavior and distribution. For example, the presence of vaginal fluid in the vagina can lead to a change in gel viscosity through dilution. Despite dilution by vaginal fluid, the disclosed hydrogel still maintains suitable viscosities.

< compositions for use in >

Besides the importance of a vaginal formulation providing a favorable distribution over the vaginal mucosa, the disclosed compositions' enhanced stability allows an effective release time for the active ingredients. The disclosed gels have an extended residence time due to their mucoadhesive and thermosensitive properties. Extended residence can permit reductions in the frequency of administration required for an effective treatment and enhance patient compliance. In some embodiments, the disclosed gels may provide a controlled-release mechanism for delivery progesterone to a subject. Controlled-release systems often reduce the amount of drug needed to achieve a desired therapeutic effect, possibly reducing the cost of therapy. In some embodiments, the disclosed gels may liberate up to 50% of the loaded progesterone within the first four hours post-administration, and as the gradual erosion of the hydrogel continues over time, more drug will be liberated in a controlled manner providing an effective absorption and a steady progesterone tissue concentration. The disclosed compositions are also biocompatible to vaginal epithelial tissue and cause minimal or no disturbance to the vaginal flora.

**3. Composition for use in medical method**

The methods described herein include methods for the sustained intravaginal delivery of progesterone and methods for treating disorders or disease in a subject in need of treatment. In some embodiments, the disclosed methods may comprise intravaginally administering to a subject in need thereof a composition comprising glycol chitin and progesterone having a pH between 3.5 and pH 5.5, where the composition exhibits a gelation temperature between 30°C to 36°C. In general, the disclosed methods include administering the compositions disclosed above. According to the disclosed methods, the composition comprising glycol chitin and progesterone may be self-administered by the subject or may be administered by a health care provider.

Prior to administration, a compositions used in the above-described methods may be brought to or maintained at a temperature below the gelation temperature of the composition. The composition will thus remain in a flowable sol state until the time of administration, enhancing the ease of use. Upon administration and accompanying exposure to body-temperature conditions, the composition will rapidly transition to a stable, mucoadhesive gel state, ensuring extended residence time at the side of administration. The subject in the disclosed methods may be a human, and in some embodiments, the subject may have a disorder such as, for example, endometrial hyperplasia or endometrial cancer.

In some embodiments, the viscosity the gels formed by the disclosed compositions may decrease in the presence of seminal fluid. Various factors may contribute to such viscosity loss, such as extensive dilution of the composition by seminal fluid and temporary changes in local pH due to the presence of seminal fluid. Therefore, in some embodiments, the disclosed methods may include providing the subject with notice that the composition should not be administered shortly before or after intercourse, such as, for example, about one hour before or after vaginal intercourse.

The disclosure also provides methods for making the disclosed compositions comprising glycol chitin and progesterone. Preparation of glycol chitin may be carried out using glycol chitosan and an acetylating agent, such as acetic anhydride or acetic chloride. An aqueous solution of glycol chitosan may be exposed to an acetylating agent in the presence or absence of an additional solvent, such as methanol. The acetylation reaction may be carried out at a temperature between -10°C and 60°C, and preferably between 15°C and 25° C, for 0.5-72 hours, and preferably 20-40 hours. In general, the glycol chitin may be prepared according to methods described in U.S. Patent 8,445,465. Progesterone may then be dissolved in a solution of glycol chitin to yield the disclosed composition comprising glycol chitin and progesterone.

**4. Kits**

In another aspect, the disclosure provides kits for the vaginal delivery of progesterone comprising a composition including glycol chitin and progesterone as described herein and an application device capable of delivering the composition. In some embodiments, the disclosed kits may provide instructions for self-administration by a subject. In some embodiments, the disclosed kits may include instructions that the kit should not be used shortly before or after intercourse,

such as, for example, about one hour before or after vaginal intercourse. In some embodiments, the kit may be provided with multiple doses of the composition.

The applicator may be any type of applicator known to those of ordinary skill in the art suitable for vaginal delivery of pharmaceutical substances. For example, the applicator may be a plunger-type applicator. In some embodiments, the applicator may be provided in the kit pre-loaded with a composition as disclosed herein comprising glycol chitin and progesterone. In some embodiments, the applicator may be single-use.

The present disclosure encompasses multiple aspects and embodiments, some of which are illustrated by the following non-limiting examples.

EXAMPLES

**Example 1**

*Materials*: Progesterone was purchased from Spectrum (Gardena, CA, USA). Glycol chitosan (degree of polymerization ≥ 400; degree of acetylation 9.34%) and acetic anhydride (99.5%) were purchased from Sigma-Aldrich (St. Louis, MO, USA). Crinone® gel (lot no. C11124) was purchased from Watson Pharmaceutics (Morristown, NJ, USA). Gynol II® (lot.no EGFR) was purchased from Revive (Madison, NJ, USA). Porcine stomach derived Mucin type III was purchased from Sigma-Aldrich (St. Louis, MO, USA). Carbopol® 934 (Lot no. KK219KA320) was received as a kind gift from Lubrizol (OH, USA). All other chemical reagents used were of analytical grade and used without further purification.

*Cell culture:* VK2/E6E7, Human vaginal epithelium cell line (CRL-2616) was purchased from the American Type Cell Culture collection (ATCC). Keratinocyte-serum free medium (GIBCO-BRL 17005-042) was purchased from Invitrogen (Grand Island, NY, USA). VK2/E6E7 cells were routinely maintained in keratinocyte-serum free medium supplemented with 0.1 ng/ml human recombinant epidermal growth factor, 0.05 mg/ml bovine pituitary extract, 100 units/ml penicillin, and 100 μg/ml streptomycin and additional calcium chloride of 0.4 mM. 293T human embryonic kidney cells line (CRL-3216) was purchased from ATCC. Dulbecco's Modified Eagle's Medium (DMEM) (ATCC 30-2002) was purchased from ATCC. 293T were routinely maintained in DMEM supplemented with 10 % fetal bovine serum (FBS) and 100 units/ ml penicillin, and 100 μg/ml streptomycin. Cells were grown and routinely maintained in 37°C in 10-cm2 culture dishes. *Lactobacillus crispatus* was purchased from ATCC and grown in an ATCC medium 146 Lactobacillus MRS agar/broth (BD, Sparks, MD,USA) at 37°C.

**Example 2**

*Preparation of Glycol Chitin*: Glycol chitosan (0.2 g) was dissolved in 25 mL distilled water and then diluted by 25 mL methanol. Acetic anhydride was added into the glycol chitosan solution under magnetic stirring, with the amount of acetic anhydride selected based on the molar ratio of acetic anhydride to the amine groups of the glycol chitosan. After stirring the reaction for 48 hours at room temperature, the polymer was precipitated in acetone, followed by centrifugation to obtain a white solid. The polymer was then treated with sodium hydroxide solution (1 M) for 12 hours to remove O-acetylation and then dialyzed against distilled water for 3 days using a dialysis membrane (MWCO = 2 kDa). Glycol chitin was obtained by lyophilization in a powdered form. Glycol chitin was characterized by 1H NMR spectroscopy using a JNM-AL400 spectrometer (Jeol Ltd., Akishima, Japan) operating at 400 MHz. Sample was prepared by dissolving glycol chitin in $D_2O$ (1.0 wt%). The degree of acetylation (DA) was calculated from 1H NMR spectra by comparing the integrated signal area of the protons of the glucopyranosyl ring ($\delta$=3.55) with that of methyl protons of acetyl group ($\delta$=1.89). Exemplary results of glycol production procedures using different molar ratios of acetic anhydride are shown below in Table 1.

**Table 1.**

| Sample | Molar Ratio (acetic anhydride/amine) | Degree of Acetylation (%) | Yield (%) |
|---|---|---|---|
| Glycol Chitosan | n/a | 9.34 ± 2.50 | n/a |
| 1 (Reference) | 0.25 | 27.55 ± 2.30 | 82.59 |
| 2 (Reference) | 0.5 | 54.09 ± 2.43 | 80.37 |
| 3 (Reference) | 1.0 | 73.12 ± 1.37 | 73.24 |
| 4 | 3.0 | 84.45 ± 1.28 | 78.61 |
| 5 | 5.0 | 86.42 ± 3.07 | 77.39 |
| 6 | 10.00 | 91.59 ± 3.10 | 79.46 |

*Preparation of compositions including Glycol Chitin and Progesterone:* Glycol chitin solution was prepared according to the modified cold method. Briefly, glycol chitin (degree of acetylation 90%) was slowly added to cold citrate phosphate buffer (4°C, 0.1 M, pH 4.2) under gentle mixing and allowed to dissolve completely for 24 hours at 4°C. Compositions comprising glycol chitin and progesterone were prepared by adding progesterone into a solution of glycol chitin. For example, progesterone was added into a 5 wt% glycol chitin solution (pH 4.2) in a concentration in excess of progesterone solubility and stirred for 24 hours at 4°C. The final progesterone concentration in glycol chitin solution was 1.0 mg/mL. Osmolarity of the resulting composition was 358.16 ± 6.5 mmol/kg.

To test the solubility of progesterone in glycol chitin solutions, progesterone was dissolved in a glycol chitin solution (for example, 1-5 wt% glycol chitin) and stirred for 24 hours at 4°C. The clear glycol chitin-progesterone solution collected from centrifugation (12,000 RPM, 4°C) was used to determine the amount of progesterone solubilized into glycol chitin employing reverse-phase HPLC (Agilent Chemstation for LC/MS system, CA, USA) equipped with a C18 column at 254 nm. A mixture of methanol/water (80:20; v/v) was used, as the mobile phase at a flow rate of 1 mL per minute. Progesterone solubility proportionally increased with increasing glycol chitin concentration reaching 148.4 ± 13.8 μg/ml in 5 wt % glycol chitin solution, which did not exceed the solubility of progesterone (~200 μg/ml) in a mixture of PEG-400/water (50:50 v/v). Fig. 1. To further increase the amount of progesterone loaded within the gel, progesterone was added to 5 wt % glycol chitin solution in a concentration that exceeded its solubility, resulting in a composition comprising 5% by weight glycol chitin and 1 mg/mL progesterone.

**Example 3**

To determine mucoadhesive property of glycol chitin, a mixture of glycol chitin and mucin was prepared at pH 4.2. The final concentrations of mucin and glycol chitin in the solution were 4 and 5 wt %, respectively. Glycol chitin and mucin were mixed for at least one hour prior to rheological evaluation. Carbopol® 934 (C934), a polymer that is known to have strong mucoadhesive properties, was used as the method standard. Rheological analyses were performed using s strdss-controlled rheometer (AR 550; TA instrument, DE, USA) equipped with 20 mm 4 degree steel cone geometry at 37°C. The linear viscoelastic region (LVR) for each sample was determined through a strain sweep measurement at a constant frequency of 1Hz. Frequency sweep analysis was performed at a range of 0.1-10 Hz within the LVR of the gel. The mean *G'* for the samples was extracted from frequency sweep spectra. Positive values of the absolute synergism parameter (*ΔG*), which is equal to the interaction between polymer and mucin, is an indicative of mucoadhesion. The following equation was used to calculate *ΔG*:

$$\Delta G = G'_{mix} - G'_p$$

Where $G'_{mix}$ and $G'_p$ are the elastic moduli for polymer-mucin mixture and the polymer alone, respectively. Since the elastic modulus of mucin ($G'_m$) is relatively low, contribution of $G'_m$ was neglected in the equation. Rheological analysis of glycol chitin showed mucoadhesive capability, demonstrated by a positive rheological synergism parameter ($\Delta G > 0$) that existed between glycol chitin and mucin under physiological pH (~4.2) shown in Figure 2.

**Example 4**

*Thermosensitive sol-gel transition of glycol chitin in vitro.* Thermo-sensitive properties of the glycol chitins were investigated by the tube inverting method and rheological studies. The tube inverting method was carried out with temperature increase of 0.2°C/min. Glycol chitin solutions with a given concentration (3-7 wt%, 1 mL) were prepared by dissolving the glycol chitins in PBS solution (pH 7.4, 0.01 M) at 4°C. The sol-gel transition temperature was determined by a flow (sol) or no-flow (gel) criterion over 30 seconds with the tube inverted. Each data point is an average of three measurements with standard deviation (mean ± SD). The sol-gel transition phase diagram obtained using this method is known to have a precision of ±1 °C.

Figure 3(a) shows a sol-gel transition phase diagram obtained by the tube inverting method. With the degree of acetylation ranging from 84.45% to 91.59%, glycol chitin solutions demonstrated a phase transition from a transparent, flowing sol state to a non-flowing transparent gel state as temperature increased (Fig. 3(a), inset), whereas no thermosensitive gelation behavior was observed for glycol chitosan and glycol chitins with a lower degrees of acetylation. The sol-gel transition temperature could be controlled from 23°C to 72°C by varying the degree of acetylation and concentrations of the glycol chitins. The phase diagram shifted to a lower temperature by increasing the degree of acetylation of the glycol chitin. An increase in the concentration of glycol chitin from 3 to 7 wt% led to a decrease in sol-gel transition temperature, which may result from higher physical cross-linking density formed by the hydrophobic interactions among acetyl groups and enhanced chain entanglement. The sol-gel transition temperature was slightly affected by adding

salts. The presence of sodium chloride, as a water-structure breaking salt, decreases the sol-gel transition temperature in typical thermogelling polymer systems of polyesters and polyphosphazenes. As the sodium chloride concentration increased to 2.5 M, the sol-gel transition temperature decreased by 3-15°C depending on the glycol chitin concentration (Fig. 3(b)).

Rheological studies were carried out to investigate the sol-gel transition behavior of the glycol chitins in response to temperature change (15-75°C), as dynamic rheometry was identified as a more reproducible and quantitative method compared to the tube inverting method. Rheological experiments were carried out by measuring changes in viscosity with a rheometer (RVDV-III+; Brookfield Instruments, USA). The temperature range was 15-75°C with a heating rate of 0.34°C/min and a fixed shear rate of 0.1/s. The reversible sol-gel transition behavior of glycol chitin in PBS solution (pH 7.4, 0.01 M, 7 wt%) was confirmed using a rotating, temperature controlled rheometer (Bohlin Advanced Rheometer, Malvern Instruments, UK). The temperature was cycled between 37°C and 4°C. The contribution of solid-like behavior (elastic modulus G') was recorded with changing temperature using a parallel plate (20 mm). Frequency was optimized to 1 Hz as determined using a frequency sweep experiment. A constant stress of 25 Pa was used for the measurement.

As shown in Fig. 3(c), the higher degrees of acetylation tested (86.42% and 91.59%) led to sharp increases in viscosity as the temperature increased. However, glycol chitosan and glycol chitins with lower degrees of acetylation at the same concentration (7 wt%) did not show an obvious increase in viscosity as the temperature increased, which coincided with the result from the tube inverting method. Moreover, a concentration dependent sol-gel transition behavior of glycol chitin was confirmed. As shown in Fig. 3(d), a sharp increase in the viscosity of glycol chitin solutions (91.5% degree of acetylation) with concentrations of 3, 5, and 7 wt% was observed at 51.80°C, 35.80°C, and 20.90°C respectively. These data demonstrated that both the degree of acetylation and concentration of glycol chitins collectively affected the sol-gel transition properties of the glycol chitins. The sol-gel transition temperatures of glycol chitin (91.59% degree of acetylation; 5 and 7 wt%) were below body temperature. In addition, unlike chitosan, gelation could be observed at relatively low concentrations and triggered only by temperature change, without the use of any chemical cross-linking agent or additives.

**Example 5**

An exemplary glycol chitin solution (91.59% degree of acetylation 7 wt%) was used to evaluate the thermogelling kinetics of glycol chitin as determined using the time for change in the elastic modulus (G') as the temperature changed using a rotating, temperature controlled rheometer. As shown in Fig. 4, the sol-gel transition of glycol chitin was fast and reversible in response to temperature. The G' showed cyclic changes as the temperature was cycled between 4°C and 37°C, matching with the reversible sol-gel transition behavior. Fast gelation is preferred for practical applications, particularly for thermogelling systems, as slow gelation in vivo may result in undesired diffusion of hydrogel precursors or bioactive molecules into surrounding tissues or failure of gel formation.

**Example 6**

To confirm the thermo-sensitive sol-gel transition in vivo, an aqueous solution of glycol chitin was injected subcutaneously into the right/left flanks of 9-week-old female nude mice. Nine-week-old female nude mice (Jackson Lab, Sacramento, CA, USA) were anesthetized by an intraperitoneal (i.p.) injection of ketamine/xylazine (300 mg ketamine combined with 20 mg of xylazine in a 4 mL volume) into each mouse (1 μL per g body weight). Glycol chitin (91.59% degree of acetylation, 7 wt%) was dissolved in 0.9% NaCl solution and 10 μL of 0.4% trypan blue solution (Invitrogen, Carlsbad, CA, USA) was added to produce glycol chitin solution with blue color. Mice were then treated with 0.2 mL of glycol chitin or 0.9% NaCl solution as a control via subcutaneous (s.c.) injection. The injection site was surgically accessed after 15 min and observed for stable gel formation at the injection site.

Upon surgically accessing the injection site 15 min after the injection, no control solution was observed at the control injection site. The sol-gel transition had occurred at the glycol chitin injection site as confirmed by observing local gelation of the glycol chitin solution as a result of the change in temperature under physiological conditions, including exposure to body temperature. Of interest, glycol chitin lost its gel-like properties and converted to a solution-like state within 10 min after opening the injection site, suggesting that the sol-gel transition of glycol chitin was reversible depending on the temperature changes. These in vivo experiments demonstrated that glycol chitin underwent the sol-gel transition under physiological conditions and this sol-gel transition was reversible depending on temperature.

**Example 7**

The macroscopic properties of glycol chitin hydrogels were studied in terms of their swelling behavior. Glycol chitin hydrogel samples (91.59% degree of acetylation, 7 wt%, 1 mL) were prepared in 5 mL vials and an excess of 3 mL PBS was gently added to the top of the glycol chitin hydrogel at 37°C. At predetermined time intervals, PBS was

fully removed from the swollen glycol chitin hydrogels, and the resulting hydrogels were weighed. The PBS was freshly replaced after every weight measurement. The weight of the swollen glycol chitin hydrogels (Ws) was measured to calculate the swelling ratio (SR). The SR of the hydrogel was calculated using the following equation, where Ws and Wi represent the weights of the swollen hydrogel samples and the initial hydrogel samples, respectively. Three triplicate experiments were performed.

$$SR = (Ws - Wi)/ Wi \times 100\%$$

Glycol chitin hydrogel reached its maximum swelling around 8 hours, with a swelling ratio of 170% while maintaining its swellings state up to 24 hours without showing any apparent changes.

**Example 8**

To assess the effect of glycol chitin concentration on the gelation temperature of compositions comprising glycol chitin and progesterone, a series of compositions were prepared at pH 4.2 having a constant concentration of progesterone (1 mg/ml) and 90.0% ± 2% degree of acetylation for the glycol chitin. The concentration of glycol chitin in the compositions was varied in the range of 3.0 to 7.0 wt%, and each composition was tested to determine the gelation temperature (see Table 2 below and Fig. 5).

**Table 2.**

| *Glycol Chitin (wt%)* | 3.0 | 3.5 | 4.0 | 4.5 | 5.0 | 5.5 | 6.0 | 7.0 |
|---|---|---|---|---|---|---|---|---|
| *Gelation Temp (°C)* | no gelation | 44.5 | 40.0 | 35.5 | 34.2 | 30.5 | 29.8 | 24.0 |

The results indicate that glycol chitin-progesterone compositions prepared with 1 mg/ml progesterone, glycol chitin having a degree of acetylation of 90.0% ± 2%, and glycol chitin concentrations between 4.5 and 6.0 wt% have gelation temperatures at pH 4.2 in the preferred range of between about 30°C and about 36°C for mucosal vaginal application. Comparable compositions having glycol chitin concentrations between below 4.5 wt% and above about 6.0 wt% either formed no gel or exhibited gelation temperatures outside of the useful range for vaginal administration (i.e., above normal body temperature or approaching room temperature, which might complicate administration of the compositions and/or handling of the compositions prior to administration).

**Example 9**

To assess the effect pH on the gelation temperature of compositions comprising glycol chitin and progesterone, a series of compositions having 1 mg/ml progesterone and 5.0 wt% glycol chitin with 90.0% ± 2% degree of acetylation (expressed as an average range based on triplicate measurements) were prepared in citrate-phosphate buffer at different pH values. Normal healthy vaginal pH is slightly acidic, in the range of about pH 3.5 to about pH 5.5, with an average value of approximately 4.2. Each composition was tested to determine the gelation temperature (see Table 3 below and Fig. 6).

**Table 3.**

| *pH* | 3.2 | 3.8 | 4.8 | 5.2 | 5.8 |
|---|---|---|---|---|---|
| *Gelation Temp (°C)* | 36.5 | 35 | 33 | 31.5 | 30 |

The results indicate that glycol chitin-progesterone compositions prepared with 1 mg/ml progesterone and 5.0 wt% glycol chitin having a degree of acetylation of 90.0% ± 2% have suitable gelation temperatures for mucosal vaginal application in a pH range spanning at least pH 3.8 to pH 5.8. The observed gelation temperature at pH 3.2 was slightly outside the preferred gelation temperature range of 30°C to 36°C, but pH 3.2 is also slightly more acidic than normal average vaginal pH values.

**Example 10 (Reference Example)**

A series of compositions comprising 1 mg/ml progesterone and glycol chitin with 80.0% ± 2% degree of acetylation were prepared at pH 4.2, each having a different wt% of glycol chitin. Each composition was then tested to

determine its gelation temperature using a stress-controlled rheometer (AR 550; TA Instrument, DE, USA). The rheometer was equipped with 20 mm, 4° steel cone geometry. Frequency and oscillatory stress were maintained with constant values of 1 Hz and 5 Pa, respectively. The temperature sweep analyses were conducted in the range of 15°C to 50°C with a heating rate of 1°C/minute. The gelation point was identified as the crossover point between G' (elastic modulus) and G" (viscous modulus). The following wt% of glycol chitin were tested: 3.0%, 4.0%, 5.0%, 6.0%, 8.0%, and 10.0%.

The results indicate that glycol chitin-progesterone compositions prepared at pH 4.2 with 1 mg/ml progesterone and glycol chitin having an 80% ± 2% degree of acetylation do not have gelation temperatures within the preferred range of about 30°C to about 36°C at the concentrations of glycol chitin tested. The compositions comprising 3.0%, 4.0%, 5.0%, and 6.0% glycol chitin did not form a gel under the pH and temperature conditions tested (Fig. 7(a)-(d)). The compositions comprising 8.0% and 10.0% were found to form a gel at approximately 48°C and 44°C, respectively (Fig. 7(e)-(h). Accordingly, a degree of acetylation greater than 80% ± 2% may be needed to achieve gelation properties suitable for vaginal administration at the progesterone and glycol chitin concentrations tested.

**Example 11**

Two compositions comprising 5 wt% glycol chitin with 90.0% ± 2% degree of acetylation were prepared at pH 4.2, the first having no progesterone and the second having 1 mg/mL progesterone. The gelation temperatures of the two compositions were determined using a stress-controlled rheometer as described above. The composition comprising progesterone demonstrated a slight increase in gelation temperature (34.3°C ± 1.6°C) compared to the composition lacking progesterone (30.9°C ± 1.6°C). *See* Fig. 7.

Additional experiments were conducted to assess the amount of progesterone that could be stably incorporated into a gel prepared from a composition comprising glycol chitin and progesterone as disclosed. In the disclosed compositions, progesterone is thought to complex with glycol chitin in micellar clusters, with the progesterone molecules dispersed in micellar spaces proximate to the hydrophobic groups of the glycol chitin molecules. *See* Fig. 15. A series of compositions comprising 5 wt% glycol chitin having a 90% ± 2% degree of acetylation were prepared at pH 4.2, each having a different concentration of progesterone. Each composition was incubated at room temperature for one week and then evaluated for phase separation, which indicates instability of the composition due to separation/precipitation of progesterone from the glycol chitin.

**Table 4**

| Progesterone (mg/ml) | 1.0 | 1.2 | 1.6 | 2.0 | 4.0 | 8.0 |
|---|---|---|---|---|---|---|
| 1 week at room temp. | Single phase; no separation | Single phase; no separation | Single phase; no separation | Phase separation | Phase separation | Phase separation |

The results showed that compositions having progesterone concentrations of 1.0, 1.2, and 1.6 mg/ml remained in stable solution form with no progesterone precipitation after one week, while those having higher progesterone concentrations of 2.0, 4.0, and 8.0 mg/ml exhibited phase separation indicative of progesterone egress and unstable formulations. Accordingly, progesterone concentrations less than about 2.0 mg/ml may be preferable for use in the disclosed compositions.

**Example 12**

*Rate of Gelation:* To determine the time required for compositions containing glycol chitin and progesterone to transform to a gel state at body temperature, a time sweep analysis was conducted using a composition comprising 1 mg/mL progesterone and 5 wt% glycol chitin with 90.0% ± 2% degree of acetylation at pH 4.2. Time sweep analysis was set for 10 minutes at a constant frequency of 1 Hz in addition of 5 Pa oscillatory stress. The sample was set to equilibrate for 5 minutes at 15°C in the conditioning step to assure a liquid state of before initiating the time sweep. At the onset of time sweep, the temperature was raised to 37°C and gelation time was recorded. As shown in Figure 9, a stable and saturated gel formed within seconds after exposure of the composition to vaginal temperature (37°C).

*Mechanical characteristics of progesterone-glycol chitin hydrogels:* To determine the mechanical spectra of progesterone-glycol chitin gel, an oscillatory stress sweep step was conducted using a stressed-controlled rheometer (AR550; TA instrument, DE, USA) equipped with 20 mm 4 degrees steel cone geometry. 150 microliters of sample was placed on the Palter plate using a positive displacement pipette (Gilson, WI, USA). The sample was allowed to equilibrate for 5 minutes at 37°C. An oscillatory stress step was conducted between the ranges of 0.5-1000 Pa. Frequency was kept constant at 1 Hz.

The oscillatory stress sweep was performed comparing samples of a progesterone-glycol chitin composition (1 mg/mL progesterone, 5 wt% glycol chitin 90.0% ± 2% degree of acetylation, pH 4.2) and 8% Crinone to compare the strengths of the two gels in vaginal like environment. Dynamics of progesterone-glycol chitin hydrogel rheological properties at 37°C revealed a solid-like behavior with a rigid microstructure under oscillatory stress ranging from 0.5 to 1000 Pa (*See* Fig. 10(a)). The critical oscillatory stress (COS), the maximum stress at which the linear value of G' is disturbed, was obtained from the stress step spectra. In terms of COS, the progesterone-glycol chitin gel formed a 1.5-fold firmer gel than 8% Crinone (Fig. 10(b)). In addition, the progesterone-glycol chitin composition had a dominant elastic property (G' > G") during frequency sweep analyses (Fig. 10(c)) and indicated a tan $\delta$ = G' / G" < 1 in the frequency region of 0.1 - 10 Hz at 37°C, as shown in Figure 10(d). The frequency range used here is commonly used for the evaluation of vaginal formulations.

**Example 13**

To determine flow behavior of progesterone-glycol chitin hydrogels, a shear step analysis was conducted in the biologically relevant shear range of 0.1-100 s-1, resembling shear ranging from passive seeping of vaginal epithelium all the way to shear during coitus. Frequency was kept constant at 1 Hz. 150 microliters of a sample containing 1 mg/mL progesterone and 5 wt% glycol chitin having 90% degree of acetylation, pH 4.2, was placed on the Palter plate using a positive displacement pipette. The sample was allowed to equilibrate for 5 minutes at 37°C prior initiation of the shear step. In addition, to determine rheological behavior after dilution with fluids that present in the vagina, viscosities and the effect of dilution on gelation were evaluated. Vaginal fluid simulant (VFS) of pH 4.2 and seminal fluid simulant (SFS) of pH 7.7 were prepared as shown in Figure 16. Gels were diluted with VFS in a biologically relevant ratio of (1:4; VFS:progesterone-glycol chitin gel) and (1:6; VFS:progesterone-glycol chitin gel). A fresh set of samples were prepared and diluted with SFS in a biologically relevant ratio of (1:1; SFS:progesterone-glycol chitin gel). For the measurement of gel behavior following dilution, gel samples were left on the rheometer platform to equilibrate for 60 seconds prior being mixed with either VFS or SFS. Steady shear viscosity step and time sweep analyses were conducted as described earlier.

Viscosity profiles showed that progesterone-glycol chitin hydrogel presents a pseudoplastic non-Newtonian behavior (shear thinning). Under the influence of relevant biological fluids, the hydrogel showed a slight decrease in viscosity under dilution with VFS (Figure 11(a)). However, the viscosity of the progesterone-glycol chitin hydrogel was significantly decreased after the dilution with a SFS (Figure 11(b)). Time sweep analysis also showed destabilized viscoelastic moduli of progesterone-glycol chitin hydrogel and a reduction in the magnitude between G' and G" when diluted with SFS (Figure 11(d)). On the other hand, progesterone-glycol chitin hydrogel diluted with VFS clearly showed stable gel properties with constant viscoelastic moduli (Figure 11(c)).

**Example 14**

*In vitro progesterone release.* To estimate the residence time and study the mechanisms of in vitro progesterone release from progesterone-glycol chitin hydrogel, the change of gel weight as function of time following VFS exposure was measured. One mL of progesterone-glycol chitin composition (1 mg/mL progesterone, 5 wt% glycol chitin with 90% degree of acetylation, pH 4.2) was placed into replicate screw capped vials and incubated at 37°C. Following gel formation, 0.75 mL of VFS was immediately placed over the gels. Vials were then placed on the platform of a MaxQ 4450 Benchtop Incubating Shaker (Thermoscientific, IA, USA). The incubation temperature was maintained at 37°C. Samples were subjected to a rotation speed of 70 rpm. At predetermined time intervals (5, 10, 15, 20, 25, 30, 40, 50, 60, 120, 180, and 240 minutes), the VFS was removed by pipette and gels were weighed using a AG104 analytical balance (METTLER TOLEDO, Columbus, OH). The amounts of VFS removed at each time were used for further analysis of the percentage of progesterone released from the gel. To analyze the amount of progesterone release, progesterone was extracted from VFS using diethyl ether. Briefly, in a fume hood, 3 ml of diethyl ether was added to each VFS sample. The samples were vortexed for 1 minute at room temperature and then placed in -20°C until complete freezing of the aqueous phase occurred. The organic layer was then removed and the samples were kept in a fume hood until complete evaporation of the organic phase. Samples were then suspended in 80% v/v ethanol and the amount of progesterone was measured at 250 nm on a Varian Cary 400 Bio UV-visible spectrophotometer (BioTech, MD, USA). Quantitative determination of progesterone was conducted using a linear calibration curve between the range of 1.96-62.5 $\mu$g/ml and a 0.99 correlation coefficient.

Progesterone-glycol chitin hydrogels exhibited a twenty percent increase in weight in the first 30 minutes of VFS exposure, followed by a weight decrease that reached 50% within four hours of exposure to VFS (Figure 12(a)). During the same time, 50% of progesterone was released from the hydrogel (Figure 12(b)).

To confirm the bioactivity of progesterone released from progesterone-glycol chitin hydrogel, the ability of the hydrogels to induce progesterone receptor signal transduction pathways was tested. Briefly, 239T cells were seeded in

a density of 1.5 x $10^6$ cells/well in 6-well plates. A plasmid containing progesterone responsive elements (PRE) fused to firefly luciferase (Cignal reporter assay kit, QIAGEN), which served as a progesterone reporter (PR) was transfected into the 239T cells (500 ng/well). Transfection was facilitated using FuGENE® 6 Transfection Reagent (Promega, WI, USA). At 24 hours post-transfection, cells were treated with free progesterone (100 nM), progesterone-glycol chitin hydrogel, or no treatment for 18 hours. Luciferase activity was measured using Dual-Luciferase Reporter Assay System (Promega, WI, USA) according to manufacturer's instructions. Results were represented as Luciferase activity normalized to Renilla luciferase, which acts as an internal control. Progesterone released from the hydrogel composition showed comparable activity with free progesterone and also demonstrated a significant increase in reporter activity compared to untreated and cells treated with glycol-chitin hydrogel lacking progesterone (Figure 12(c)).

**Example 15**

*In vitro biodegradation and biocompatibility of glycol chitin.* Chitosan is biodegradable by lysozyme, which is widely present in the human body fluids (e.g., serum, saliva, and tears) in vivo. To ascertain whether glycol chitosan and glycol chitins were biodegradable by lysozyme, viscosity changes of aqueous glycol chitosan and glycol chitin solutions were observed in the presence of lysozyme. The biodegradation of glycol chitosan and glycol chitins with various degrees of acetylation was assessed by measuring the viscosity change of the polymer solution in the presence of lysozyme from chicken egg white. Enzymatic degradation experiments were carried out at 37°C. Glycol chitosan and glycol chitins (40 mg) were dissolved in 20 mL phosphate-buffered saline (PBS, pH 7.4, 0.01 M) solution and separately and incubated in a shaking bath (Series BS-21; Lab companion, Korea) at 37°C for 30 minutes. Lysozyme was added with a final concentration of 55 μg/mL. The viscosity change of the polymer solution was measured by a Schott-Gerate automatic viscometer (AVS350) as a function of time. The results are representative of triplicate experiments and are presented as the mean value with standard deviation (mean ± SD).

As shown in Fig. 13(a), the viscosity of glycol chitin solutions decreased to 67-87% of initial viscosity after 90 minutes, while the viscosity of the glycol chitosan solution decreased slightly to 89% of initial viscosity due lysozyme-catalyzed biodegradation. The glycol chitins exhibited a larger decrease in viscosity than that of glycol chitosan at all time points, indicating that the glycol chitin structure, an N-acetylated form of glycol chitosan, had better biodegradability than glycol chitosan, probably due to the higher content of N-acetyl glucosamine units that are more susceptible to lysozyme. The extent of lysozyme-catalyzed degradation of glycol chitins was proportionally dependent on the degree of acetylation.

To investigate whether glycol chitins exhibit cytotoxicity, MTT-based cytotoxicity assays were performed with HeLa cells. HeLa (human cervical cancer) cells were purchased from the American Type Cell Culture Collection (CCL-2, ATCC, Manassas, VA, USA). The cells were cultured in Eagle's minimum essential medium (EMEM, ATCC) supplemented with 10% fetal bovine serum (Denville, Metuchen, NJ, USA) under 5% CO2 in a humidified atmosphere. The cells were seeded into a 96-well tissue culture plate (Costar, Corning, NY, USA) at a density of $1\times10^4$ cells/well and incubated in 100 μL of EMEM/well overnight. Glycol chitins with various degrees of acetylation were dissolved in PBS and serially diluted with PBS. Based on the degree of acetylation, we divided the samples into three different groups, which are group I (glycol chitosan, and glycol chitin with 27.55% degree of acetylation), group II (glycol chitins with 54.09% and 73.12% degrees of acetylation, respectively), and group III (glycol chitin with 91.59% degree of acetylation). The cells were fed with 100 μL culture media containing a serial dilution of the glycol chitins and cultured for 72 h. Then, 20 μL MTT (3-(4,5-dimethylthiazole-2-yl)-2,5-diphenyl tetrazolium bromide) (Invitrogen, Gaithersburg, MD, USA) was added to each well of the plate with a final concentration of 50 μg/mL in culture media solution. The plates were incubated in a cell culture incubator at 37°C for 4 hours. After removing the culture media, formazan crystals were completely dissolved in 100 μL DMSO and the absorbance was measured using a microplate reader (Spectramax 250, Molecular Device Inc., Sunnyvale, CA) at a wavelength of 540 nm. The relative cell viability (%) was calculated from [ab] test/[ab] control, which refers to cells cultured in media with and without glycol chitin, respectively. The experimental results were obtained from the average values measured from three independent experiments in triplicate.

The in vitro cytotoxicity test revealed that the glycol chitins exhibited low cellular toxicity to the cultured cells, showing at least 70% of cell viability following exposure to the highest polymer concentrations tested (Fig. 13(b)). In detail, group I showed about 74 ± 3.3% and 70 ± 3.7% cell viability at 250 μg/mL, and the cell viability increased gradually up to 88 ± 8.1% and 96 ± 5.6% at 15.6 μg/mL. Interestingly, group II demonstrated a much lower toxicity profile compared to that of group I with cell viabilities of 99 ± 10.4% and 98 ± 7.1% at 250 μg/mL and the viability of group II was over 100% at 15.6 μg/mL. In contrast, group III showed cell viabilities of 89 ± 2.9% and 92 ± 1.9% at 250 μ/mL and 15.6 μg/mL respectively, which decreased slightly compared to that of group II. The increase in degree of acetylation gradually reduced cytotoxic effects toward cultured cells. The results indicate the glycol chitins are generally biologically safe and non-toxic.

## Example 16

*In vitro biocompatibility of compositions comprising progesterone and glycol chitin.* To assess the safety and toxicity of progesterone-glycol chitin compositions for a potential vaginal application, cellular and microbial toxicity of glycol chitin and progesterone-glycol chitin compositions to human vaginal epithelial cells and normal, physiological vaginal flora were measured by MTT, MTS, and clonogenic assays. First, to determine glycol chitin biocompatibility with vaginal epithelium, human VK2/E6E7 cells were seeded in 96-well plates at a density of $5x10^3$ cells/well and incubated for 24 hours in a volume of 100 μl media/well. Serial dilutions of glycol chitin in citrate-phosphate buffer (0.1M, pH 4.2) were added to the cells and incubated for another 24 hours. After incubation, 20μl of 2.5mg/ml MTT 3-(4,5-dimethylthiazol-2-yl)-2,5-diphenyltetrazolium bromide (Invitrogen, OR, USA) in PBS was added to the cells. The cells were incubated with MTT for 4 hours 37°C. MTT solutions were completely removed and 100 μl DMSO was added to solubilize formazan crystals. Absorbance was measured at 540 nm using Spectramax 250 microplate reader (Molecular device, CA, USA). Percentage cell viability was calculated from (OD540 glycol chitin-treated cells / OD540 untreated control cells) x 100. MTT assays showed no toxicity in human vaginal epithelial cells (VK2/E6E7) after exposure to glycol chitin, ranging from 84.4% ± 15.8 to 107% ± 22.3 viability when compared to untreated control cells (Fig. 14(a)).

Next, to assess the effect of glycol chitin on the sustainability of an intact, normal vaginal flora, the viability of *Lactobacillus crispatus* after the exposure to glycol chitin was determined. Bacteria were seeded in a density of $10^7$ colony forming units (CFU) / 100 μl in 96-well plates and incubated with 100 μl serial dilutions of glycol chitin (90% degree of acetylation, pH 4.2) at 37°C for 24 h. After 24 h incubation, a 20 μl MTS [3-(4,5-dimethyl thiazole-2-yl)-5-(3-carboxymethoxyphenyl)-2-(4-sulfonyl) 2H-tetrazolium (Promega, Madison, WI, USA) reagent was added to each well. Bacterial viability was determined by measurement absorbance at OD490 using Spectramax 250 microplate reader (Molecular device, CA, USA). Percent viability was calculated from (OD490 glycol chitin-treated cells/ OD490 untreated cells) x100. Since lactobacilli play a crucial role in protecting the vaginal environment from pathogens acting as a gatekeeper for the vaginal ecosystem, lactobacillus viability was tested following exposure to various glycol chitin compositions. Glycol chitin did not impact viability of lactobacillus showing (%) viability ranging from 95.6 ± 3.7 to 106.4 ± 1 (Figure 14(b)) and was therefore considered non-toxic to the vaginal flora.

Finally, clonogenic assays demonstrated that the number of vaginal epithelial VK2/E6E7 cell colonies following exposures to glycol chitin and progesterone-glycol chitin was comparable results obtained with untreated cells. Human VK2/E6E7 cells were seeded in 6-well plates (Transwell, Costar, NY, USA) in a density of 2000 cells/well. Cells were incubated in 2 ml Keratinocyte-Serum Free medium until complete cells attachment. Cells were treated with glycol chitin (5 wt%, 90% degree of acetylation), progesterone-glycol chitin (200μg progesterone, 5 wt% glycol chitin with 90% degree of acetylation), 200μg progesterone, Gynol II®, or 8% Crinone® 200μg, which were all added to a 0.4 μm polyester transwell membrane. In addition, one group of cells was left untreated and served as a blank (negative control). Cells were exposed equally to all treatments for 24 hours. Transwells were then removed and cells were cultured for additional 7 days until the formation of visible colonies. Colonies were then fixed for at least 30 minutes with a 6% glutaraldehyde and 0.5% crystal violets mixture. Wells were rinsed under running tap water and numbers of visible colonies were quantified. Blue-colored-colonies were quantified with ImageJ 1.44o software (N.I.H., Bethesda, MD, USA) (size range of 2 ~ 200μm). Although there was a slight decrease in the number of VK2/E6E7 colonies in the progesterone-glycol chitin treated group, colony formation was significantly reduced after treatment with Crinone® or Gynol II® compared to GC-P4 (Fig. 14(c)).

## Example 17

*Murine endometrial cancer model based on patient-derived endometrial cancer xenografts (PDECXs).* PDECXs retain pathological and molecular characteristics of the original patient-derived tumor specimen, and the mouse model based on direct orthotopic transplantation of PDECX tissues to the mouse uterus resembles and maintains human tumor characteristics through serial transplantation, including tumor grade, histopathological features, and hormone receptor (estrogen, progesterone) expression.

Tissue samples were collected under IRB-approved protocols from patients undergoing surgery for endometrial cancer. Tissues underwent routine histopathological examination before further laboratory processing. Each tissue specimen was mechanically separated into multiple pieces, and such pieces were then surgically implanted into the horns of the bicorn uterus of nude (nu/nu) female mice. Tissues were propagated through multiple generations of animals (G1-G3). Following implantation, animal body weights and abdominal circumferences were monitored once per week. The PDECX tissues developed orthotopic endometrial tumors in the mouse uterus, as well as distant metastases in tissues such as liver, spleen, lung, inguinal lymph node, axillary lymph node, superficial cervical lymph node, intestine, and omentum. Uterine and tumor tissue samples were sectioned and stained for estrogen receptor and progesterone receptor and by hematoxylin/eosin staining using standard microtome sectioning, deparaffinization, hydration, and antigen retrieval techniques. Immunohistochemical staining was performed using standard blocking, primary/secondary

antibody, and visualization techniques. PDECX-derived tumor histological characteristics and receptor expression were consistent with matched original patient samples. Such models may be used for preclinical drug screening and may assist rapid and economical translation of new drugs and drug delivery systems in the clinic.

**Example 18**

*In vivo biocompatibility and safety of compositions comprising progesterone and glycol chitin.* To evaluate the safety and efficacy of progesterone-glycol chitin compositions following vaginal administration in vivo, 6-8 week old Nu/Nu female mice were repeatedly treated with a progesterone-glycol chitin composition (1 mg/ml progesterone, 5 wt% glycol chitin with 90.0% ± 2% degree of acetylation, pH 4.2), Gynol II® (positive control), or phosphate buffered saline (PBS) (negative control), which were applied to the vaginal tract. Mice were obtained from Jackson Laboratory (Bar Harbor, ME USA). Mice were divided into three groups (n=2) and anesthetized by IP injection with Xylazine-Ketamine at dose of 0.1ml / 10 g. Mice then received vaginal sample administration once per day for 3 consecutive days. The animals were sacrificed 24 hours after the last dose, and vaginal tissues were trimmed and fixed in 10% formalin for 48 hours. Tissues were dehydrated in 70 % ethanol and send to Associated Regional and University Pathologists (ARUP) at the University of Utah for Hematoxylin and Eosin staining. Histology of vaginal tissues of treated and control animals were examined. Histological examinations were conducted by two independent pathologists using light microscopy at the Department of Pathology, University of Utah. The tests revealed that administration of progesterone-glycol chitin compositions to murine vaginal epithelium showed negligible toxicity and non-significant, transient signs of acute inflammation when compared to control animals group treated with PBS. In contrast, Gynol II®, a known irritant to vaginal epithelium caused chronic inflammation to murine vaginal tissues, massive death of squamous cells, and a significant increase in neutrophils.

To determine the efficacy of progesterone-glycol chitin compositions in vivo, we utilized an estrogen-induced endometrial hyperplasia (EH) mouse model. 6-8 week old female Nu/Nu mice were divided in to three groups and anesthetized by IP injection with Xylazine-Ketamine at dose of 0.1ml / 10 g. Estrogen was administered to mice in all groups by subcutaneous implantation of an estrogen dosage pellet (0.9 mg estrogen per pellet) to induce Endometrial hyperplasia (EH). One group of mice did not receive estrogen therapy and served as disease-negative control. After four weeks of estrogen treatment and confirmation of EH through histology, one group of animals, which received estrogen therapy, received daily intravaginal treatment with a progesterone-glycol chitin composition (1 mg/ml progesterone, 5 wt% glycol chitin at 90.0% ± 2% degree of acetylation, pH 4.2; 60 μg/ day) for two weeks. The second group of estrogen-treated mice received no progesterone treatment and served as disease-positive control. Animals were sacrificed 24 hours after the last progesterone-glycol chitin dose, and uterine tissues were trimmed and fixed in 10 % formalin for 48 hours. Tissues were treated as mentioned earlier and stained by hematoxylin and eosin. Histological examination was conducted by two independent pathologists using light microscopy at the Department of Pathology, University of Utah. Normal, healthy, untreated mouse endometrium showed small tubular glands, some in clusters, but overall with abundant intervening stroma representative of healthy endometrium. However, endometrial samples derived from mice that were exposed to estrogen showed the development of enlarged and pseudostratified cigar-shaped nuclei in glands and architectural "cribriforming" complexity, commonly associated with cells invading the stroma in carcinomas and characteristic of complex atypical endometrial hyperplasia (CAEH). The group of mice treated with progesterone-glycol chitin following estrogen treatment showed the regression of CAEH to simple hyperplasia of the endometrium (SHE) without atypia. A clear difference in endometrial histology between mice that received estrogen and the mice that received estrogen and the progesterone-glycol chitin composition.

Thus, the disclosure provides, among other things, a safe and effective composition for vaginal delivery of progesterone. Various features and advantages of the invention are set forth in the following claims.

**Claims**

1. A composition for intravaginal delivery, the composition comprising:

    glycol chitin having a degree of acetylation of at least 80%; and
    progesterone;
    the composition has a pH from 3.5 to 5.5, and exhibits a gelation temperature from 30°C to 36°C.

2. The composition of claim 1, wherein the concentration of glycol chitin is from 3 wt% to 8 wt%.

3. The composition of claim 1, wherein the concentration of glycol chitin is from 4 wt% to 6 wt%.

4. The composition of any of claims 1-3, wherein the degree of acetylation of the glycol chitin is from 85% to 95%.

5. The composition of any of claims 1-3, wherein the degree of acetylation of the glycol chitin is from 88% to 92%.

6. The composition of any of claims 1-3, wherein the degree of acetylation of the glycol chitin is at least 85%.

7. The composition of any of claims 1-3, wherein the degree of acetylation of the glycol chitin is at least 90%.

8. The composition of any of claims 1-3, wherein the average degree of polymerization of the glycol chitin is from 100 to 10,000.

9. The composition of any of claims 1-3, wherein the average degree of polymerization of the glycol chitin is from 200 to 1,000.

10. The composition of any of claims 1-3, wherein the average degree of polymerization of the glycol chitin is from 400 to 800.

11. The composition of any of claims 1-3, wherein the composition comprises 2.0 mg/mL or less progesterone.

12. The composition of any of claims 1-3, wherein the composition comprises 1.4 mg/ml or less progesterone.

13. A composition for use in a method for the sustained intravaginal delivery of progesterone, the method comprising intravaginally administering to a subject in need thereof said composition comprising:

   glycol chitin having a degree of acetylation of at least 80%; and
   progesterone;
   wherein the composition has a pH from 3.5 to 5.5, and exhibits a gelation temperature from 30°C to 36°C.

14. The composition for use of claim 13, wherein the composition is at a temperature below the gelation temperature at the time of administration.

15. The composition for use of claim 13, wherein the subject is a human.

16. The composition for use of claim 13, wherein the subject has endometrial hyperplasia or endometrial carcinoma.

17. The composition for use of claim 13, wherein the concentration of glycol chitin is from 3 wt% to 8 wt%.

18. The composition for use of claim 17, wherein the degree of acetylation of the glycol chitin is from 85% to 95%.

19. The composition for use of claim 13, wherein the average degree of polymerization of the glycol chitin is at least 400.

20. The composition for use of claim 13, wherein the composition comprises 2.0 mg/mL or less progesterone.

21. The composition for use of claim 13, further comprising providing the subject with notice that the composition should not be administered within one hour before or after intercourse.

22. A kit for the intravaginal delivery of progesterone, comprising:

   a. the composition of claim 1, and
   b. an application device.

**Patentansprüche**

1. Zusammensetzung zur intravaginalen Verabreichung, wobei die Zusammensetzung umfasst:

   Glykolchitin mit einem Acetylierungsgrad von mindestens 80%; und
   Progesteron;

die Zusammensetzung einen pH-Wert von 3,5 bis 5,5 hat und eine Geliertemperatur von 30°C bis 36°C aufweist.

2. Zusammensetzung nach Anspruch 1, wobei die Konzentration von Glykolchitin 3 Gew.-% bis 8 Gew.-% beträgt.

3. Zusammensetzung nach Anspruch 1, wobei die Konzentration von Glykolchitin 4 Gew.-% bis 6 Gew.-% beträgt.

4. Die Zusammensetzung nach einem der Ansprüche 1-3, wobei der Acetylierungsgrad des Glykolchitins 85% bis 95% beträgt.

5. Zusammensetzung nach einem der Ansprüche 1-3, wobei der Acetylierungsgrad des Glykolchitins 88% bis 92% beträgt.

6. Zusammensetzung nach einem der Ansprüche 1-3, wobei der Acetylierungsgrad des Glykolchitins mindestens 85% beträgt.

7. Zusammensetzung nach einem der Ansprüche 1-3, wobei der Acetylierungsgrad des Glykolchitins mindestens 90% beträgt.

8. Zusammensetzung nach einem der Ansprüche 1-3, wobei der durchschnittliche Polymerisationsgrad des Glykolchitins 100 bis 10.000 beträgt.

9. Zusammensetzung nach einem der Ansprüche 1-3, wobei der durchschnittliche Polymerisationsgrad des Glykolchitins 200 bis 1.000 beträgt.

10. Zusammensetzung nach einem der Ansprüche 1-3, wobei der durchschnittliche Polymerisationsgrad des Glykolchitins 400 bis 800 beträgt.

11. Zusammensetzung nach einem der Ansprüche 1-3, wobei die Zusammensetzung 2,0 mg/mL oder weniger Progesteron umfasst.

12. Zusammensetzung nach einem der Ansprüche 1-3, wobei die Zusammensetzung 1,4 mg/ml oder weniger Progesteron umfasst.

13. Zusammensetzung zur Verwendung in einem Verfahren zur anhaltenden intravaginalen Verabreichung von Progesteron, wobei das Verfahren die intravaginale Verabreichung an einen Patienten umfasst, der dies benötigt, wobei die Zusammensetzung umfasst:

Glykolchitin mit einem Acetylierungsgrad von mindestens 80%; und
Progesteron;
wobei die Zusammensetzung einen pH-Wert von 3,5 bis 5,5 hat und eine Geliertemperatur von 30°C bis 36°C aufweist.

14. Zusammensetzung zur Verwendung nach Anspruch 13, wobei die Zusammensetzung bei einer Temperatur unterhalb der Geliertemperatur zum Zeitpunkt der Verabreichung liegt.

15. Zusammensetzung zur Verwendung nach Anspruch 13, wobei der Patient ein Mensch ist.

16. Zusammensetzung zur Verwendung nach Anspruch 13, wobei der Patient eine endometriale Hyperplasie oder ein endometriales Karzinom aufweist.

17. Zusammensetzung zur Verwendung nach Anspruch 13, wobei die Konzentration von Glykolchitin 3 Gew.-% bis 8 Gew.-% beträgt.

18. Zusammensetzung zur Verwendung nach Anspruch 17, wobei der Acetylierungsgrad des Glykolchitins 85% bis 95% beträgt.

19. Zusammensetzung zur Verwendung nach Anspruch 13, wobei der durchschnittliche Polymerisationsgrad des Glykolchitins mindestens 400 beträgt.

**20.** Zusammensetzung zur Verwendung nach Anspruch 13, wobei die Zusammensetzung 2,0 mg/mL oder weniger Progesteron umfasst.

**21.** Zusammensetzung zur Verwendung nach Anspruch 13, ferner umfassend das Bereitstellen einer Mitteilung an den Patienten, dass die Zusammensetzung nicht innerhalb einer Stunde vor oder nach dem Geschlechtsverkehr verabreicht werden sollte.

**22.** Kit für die intravaginale Verabreichung von Progesteron, umfassend:

a. die Zusammensetzung nach Anspruch 1 und
b. eine Applikationsvorrichtung.

**Revendications**

**1.** Composition d'administration intravaginale, la composition comprenant :

de la glycol chitine ayant un degré d'acétylation d'au moins 80 % ; et
de la progestérone ;
la composition ayant un pH de 3,5 à 5,5, et faisant preuve d'une température de gélification de 30°C à 36°C.

**2.** Composition selon la revendication 1, dans laquelle la concentration de la glycol chitine est de 3 % en pds à 8 % en pds.

**3.** Composition selon la revendication 1, dans laquelle la concentration de la glycol chitine est de 4 % en pds à 6 % en pds.

**4.** Composition selon l'une quelconque des revendications 1 à 3, dans laquelle le degré d'acétylation de la glycol chitine est de 85 % à 95 %.

**5.** Composition selon l'une quelconque des revendications 1 à 3, dans laquelle le degré d'acétylation de la glycol chitine est de 88 % à 92 %.

**6.** Composition selon l'une quelconque des revendications 1 à 3, dans laquelle le degré d'acétylation de la glycol chitine est d'au moins 85 %.

**7.** Composition selon l'une quelconque des revendications 1 à 3, dans laquelle le degré d'acétylation de la glycol chitine est d'au moins 90 %.

**8.** Composition selon l'une quelconque des revendications 1 à 3, dans laquelle le degré moyen de polymérisation de la glycol chitine est de 100 à 10 000.

**9.** Composition selon l'une quelconque des revendications 1 à 3, dans laquelle le degré moyen de polymérisation de la glycol chitine est de 200 à 1 000.

**10.** Composition selon l'une quelconque des revendications 1 à 3, dans laquelle le degré moyen de polymérisation de la glycol chitine est de 400 à 800.

**11.** Composition selon l'une quelconque des revendications 1 à 3, dans laquelle la composition comprend 2,0 mg/ml ou moins de progestérone.

**12.** Composition selon l'une quelconque des revendications 1 à 3, dans laquelle la composition comprend 1,4 mg/ml ou moins de progestérone.

**13.** Composition pour l'utilisation dans un procédé d'administration intravaginale prolongée de progestérone, le procédé comprenant l'administration intravaginale à un sujet ayant besoin de celle-ci de ladite composition comprenant :

de la glycol chitine ayant un degré d'acétylation d'au moins 80 % ; et
de la progestérone ;
la composition ayant un pH de 3,5 à 5,5, et faisant preuve d'une température de gélification de 30°C à 36°C.

**14.** Composition pour l'utilisation selon la revendication 13, dans laquelle la composition se trouve à une température inférieure à la température de gélification au moment de l'administration.

**15.** Composition pour l'utilisation selon la revendication 13, dans laquelle le sujet est un être humain.

**16.** Composition pour l'utilisation selon la revendication 13, dans laquelle le sujet présente une hyperplasie de l'endomètre ou un carcinome de l'endomètre.

**17.** Composition pour l'utilisation selon la revendication 13, dans laquelle la concentration de la glycol chitine est de 3 % en pds à 8 % en pds.

**18.** Composition pour l'utilisation selon la revendication 17, dans laquelle le degré d'acétylation de la glycol chitine est de 85 % à 95 %.

**19.** Composition pour l'utilisation selon la revendication 13, dans laquelle le degré moyen de polymérisation de la glycol chitine est d'au moins 400.

**20.** Composition pour l'utilisation selon la revendication 13, dans laquelle la composition comprend 2,0 mg/ml ou moins de progestérone.

**21.** Composition pour l'utilisation selon la revendication 13, comprenant en outre l'indication au sujet d'un avertissement que la composition ne devrait pas être administrée dans l'heure précédent ou faisant suite à un rapport sexuel.

**22.** Kit d'administration intravaginale de progestérone, comprenant :

a. la composition selon la revendication 1, et
b. un dispositif d'application.

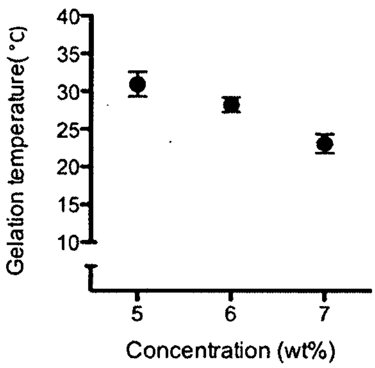

Gelation temperature( °C)
40
35
30
25
20
15
10
5
6
7
Concentration (wt%)


FIG. 1

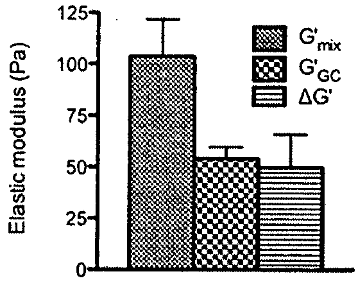


FIG. 2

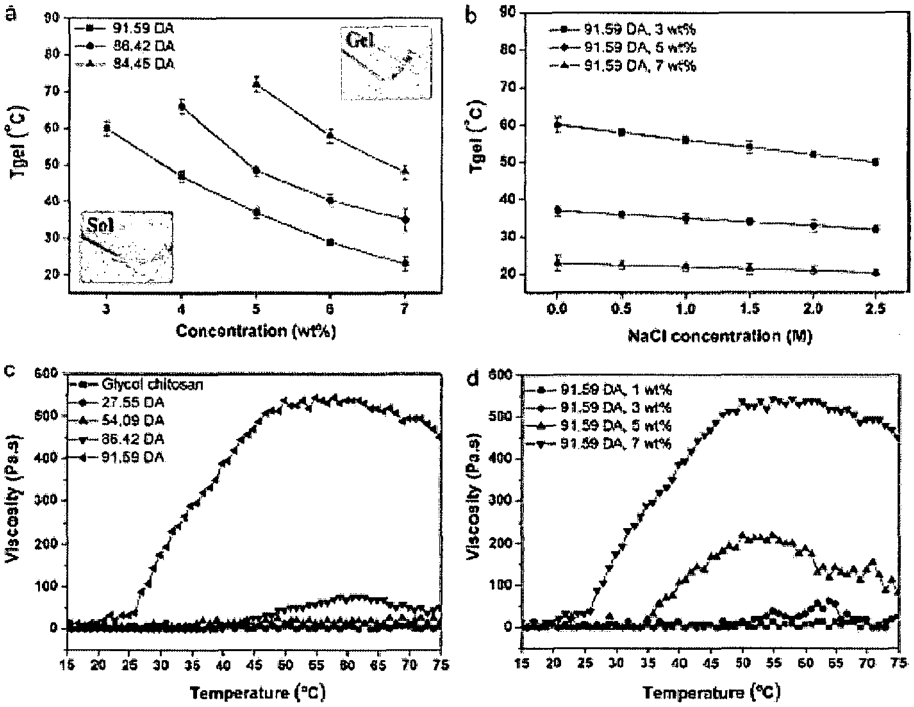
a
91.59 DA
86.42 DA
84.45 DA
Gel
Sol
Tgel (°C)
Concentration (wt%)
b
91.59 DA, 3 wt%
91.59 DA, 5 wt%
91.59 DA, 7 wt%
Tgel (°C)
NaCl concentration (M)
c
Glycol chitosan
27.55 DA
54.09 DA
86.42 DA
91.59 DA
Viscosity (Pa.s)
Temperature (°C)
d
91.59 DA, 1 wt%
91.59 DA, 3 wt%
91.59 DA, 5 wt%
91.59 DA, 7 wt%
Viscosity (Pa.s)
Temperature (°C)

**FIG. 3**

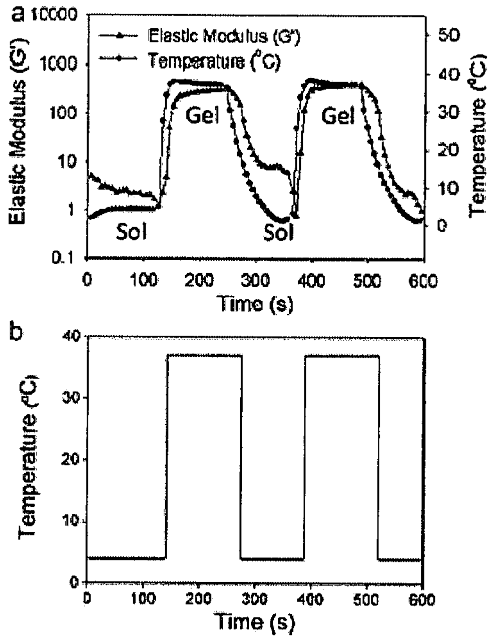


FIG. 4

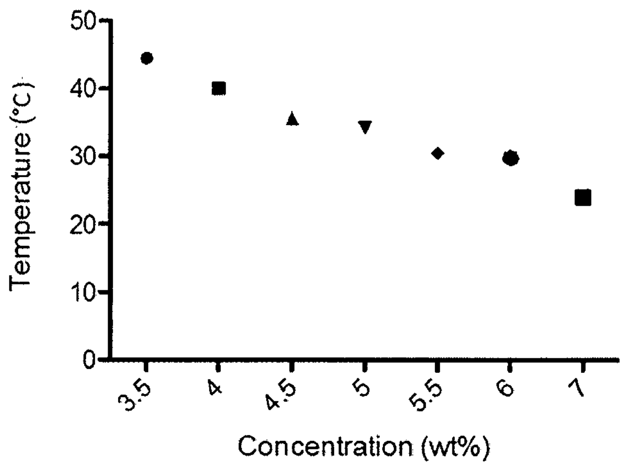


FIG. 5

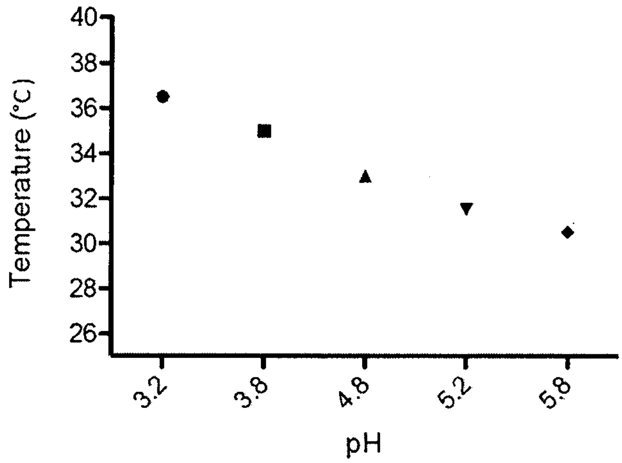


FIG. 6

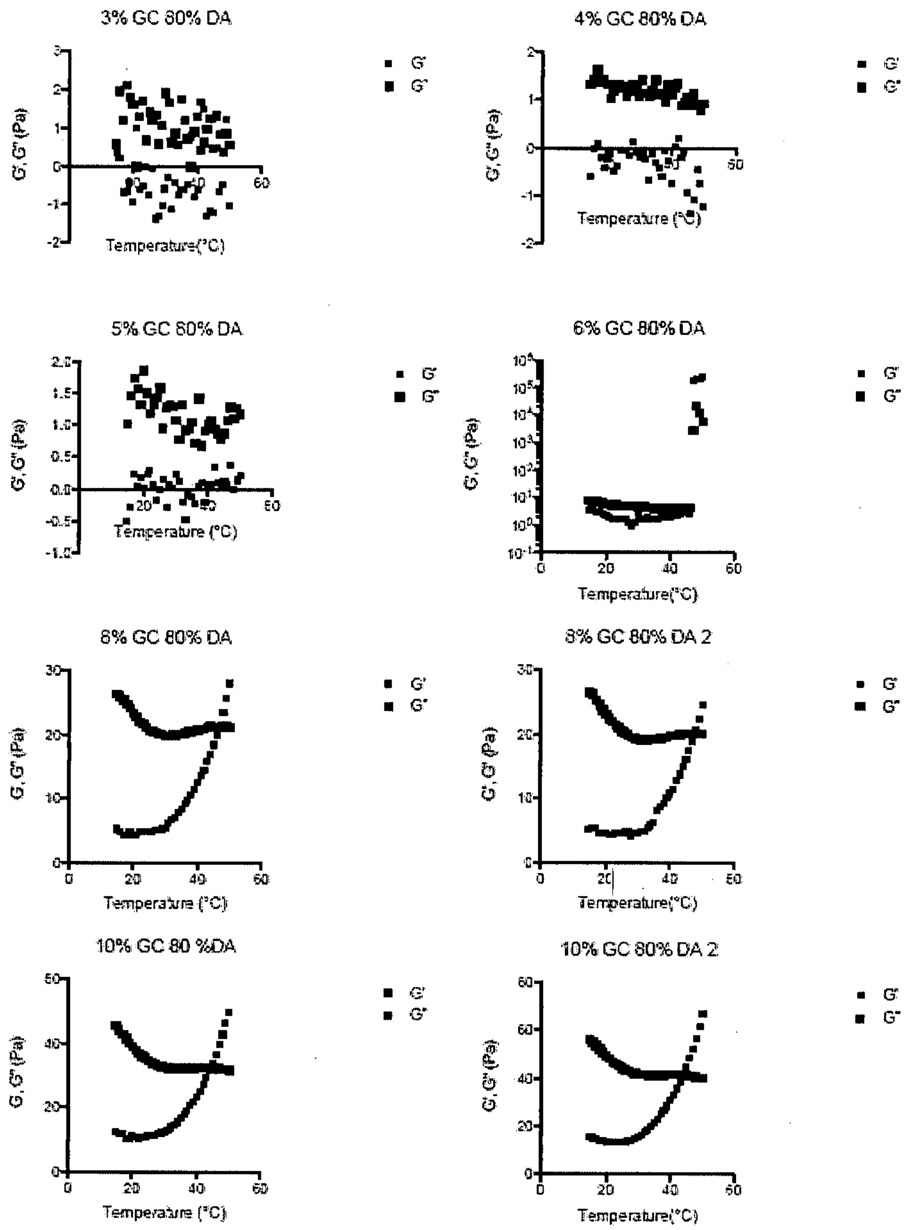
3% GC 80% DA
4% GC 80% DA
5% GC 80% DA
6% GC 80% DA
8% GC 80% DA
8% GC 80% DA 2
10% GC 80 %DA
10% GC 80% DA 2
G'
G"
G', G" (Pa)
Temperature(°C)

FIG. 7

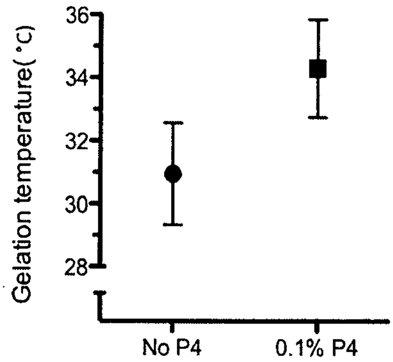


FIG. 8

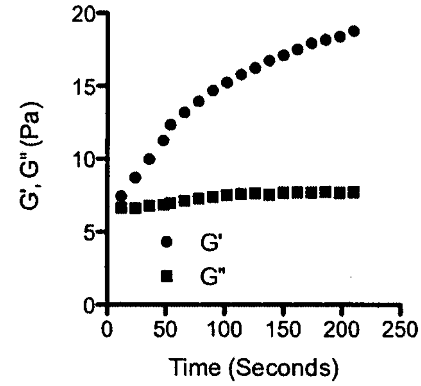


FIG. 9

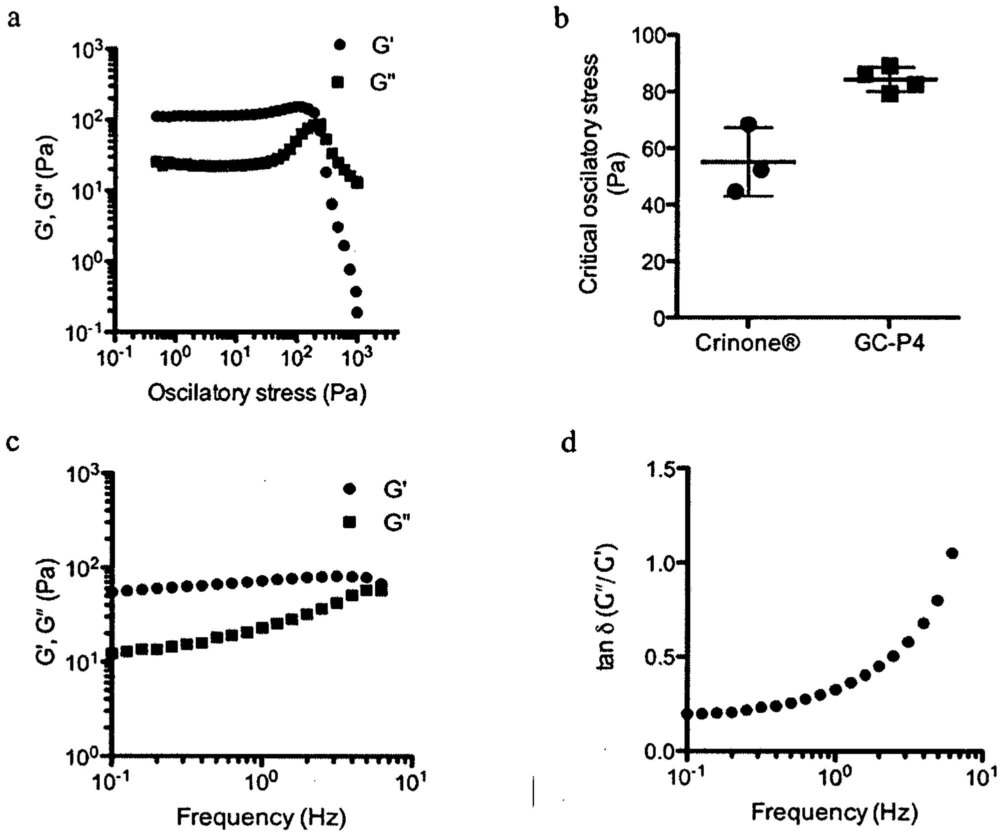

a
G'
G"
G', G" (Pa)
Oscilatory stress (Pa)
b
Critical oscilatory stress (Pa)
Crinone®
GC-P4
c
G'
G"
G', G" (Pa)
Frequency (Hz)
d
tan δ (G"/G')
Frequency (Hz)


FIG. 10

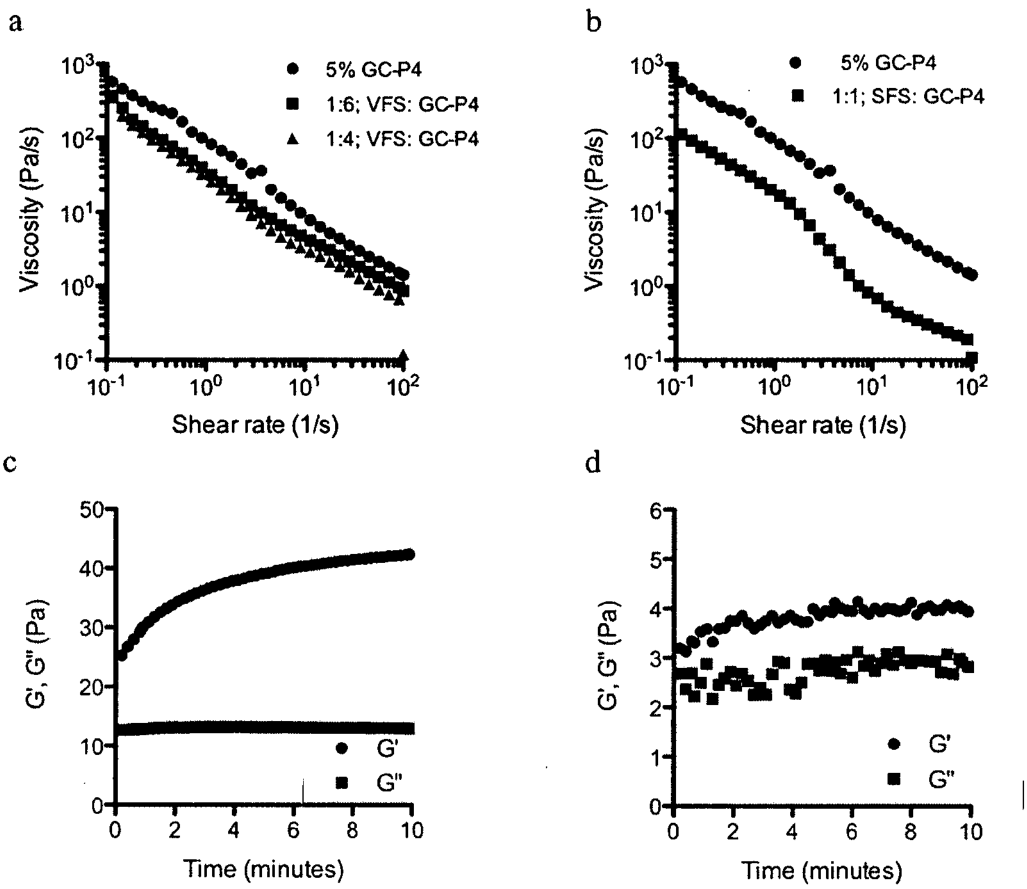
a
Viscosity (Pa/s)
Shear rate (1/s)
5% GC-P4
1:6; VFS: GC-P4
1:4; VFS: GC-P4
b
Viscosity (Pa/s)
Shear rate (1/s)
5% GC-P4
1:1; SFS: GC-P4
c
G', G'' (Pa)
Time (minutes)
G'
G''
d
G', G'' (Pa)
Time (minutes)
G'
G''

FIG. 11

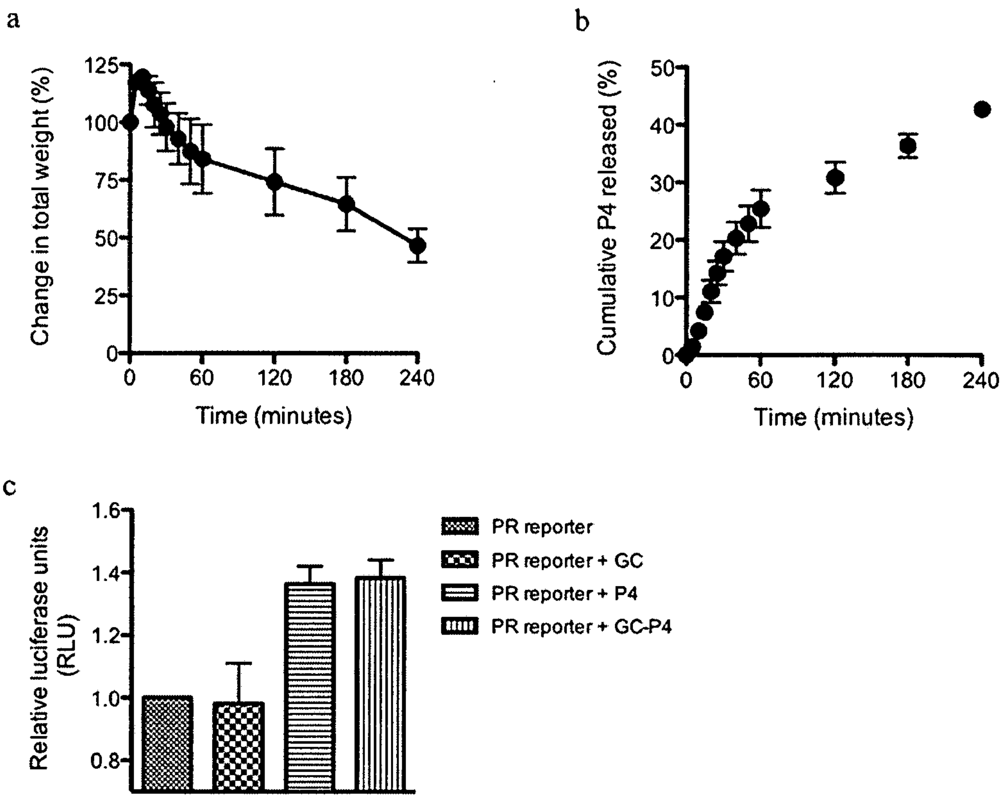
a
Change in total weight (%)
125
100
75
50
25
0
0
60
120
180
240
Time (minutes)
b
Cumulative P4 released (%)
50
40
30
20
10
0
0
60
120
180
240
Time (minutes)
c
Relative luciferase units (RLU)
1.6
1.4
1.2
1.0
0.8
PR reporter
PR reporter + GC
PR reporter + P4
PR reporter + GC-P4

FIG. 12

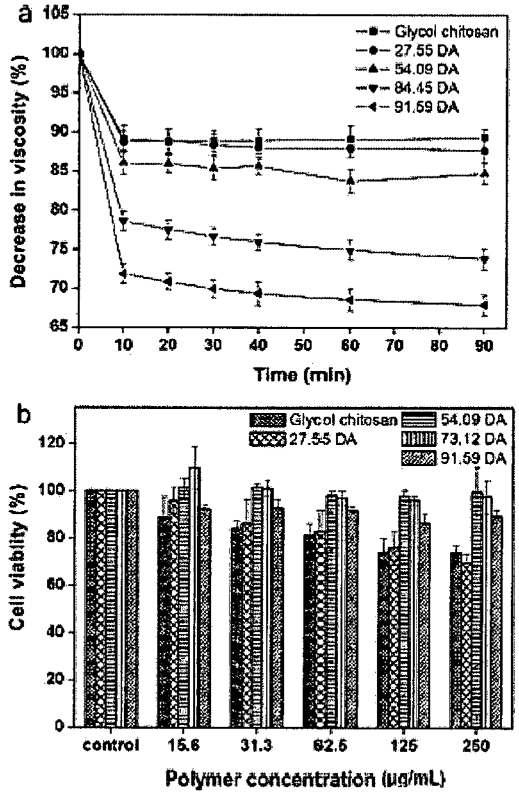
a
Decrease in viscosity (%)
Time (min)
Glycol chitosan
27.55 DA
54.09 DA
84.45 DA
91.59 DA
b
Cell viability (%)
Polymer concentration (ug/mL)
control
15.6
31.3
62.5
125
250
Glycol chitosan
27.55 DA
54.09 DA
73.12 DA
91.59 DA

FIG. 13

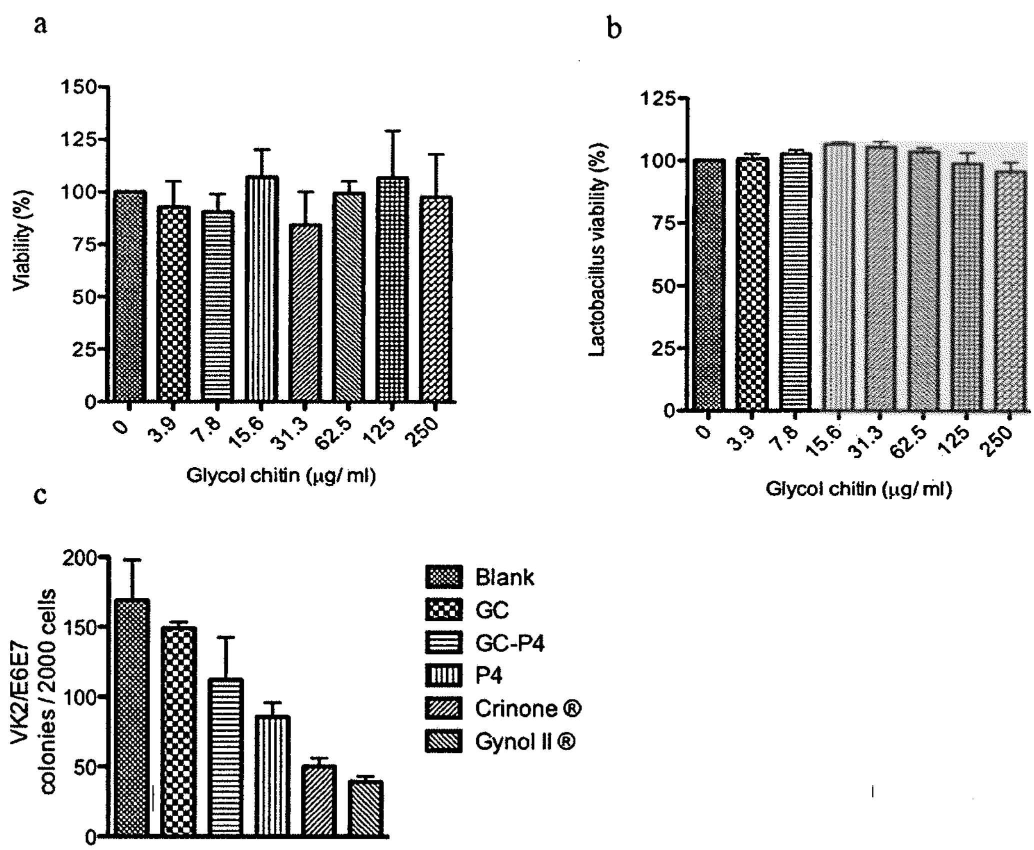
a
Viability (%)
150
125
100
75
50
25
0
0
3.9
7.8
15.6
31.3
62.5
125
250
Glycol chitin (µg/ ml)
b
Lactobacillus viability (%)
125
100
75
50
25
0
0
3.9
7.8
15.6
31.3
62.5
125
250
Glycol chitin (µg/ ml)
c
VK2/E6E7 colonies / 2000 cells
200
150
100
50
0
Blank
GC
GC-P4
P4
Crinone ®
Gynol II ®

FIG. 14

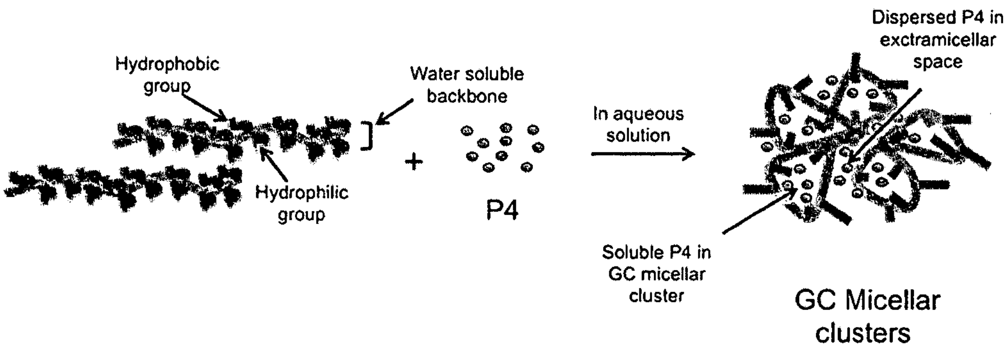
Hydrophobic group
Water soluble backbone
Hydrophilic group
+
P4
In aqueous solution
Dispersed P4 in exctramicellar space
Soluble P4 in GC micellar cluster
GC Micellar clusters

FIG. 15

**Vaginal Fluid Simulant (VFS)**

| Component | Concentration (mM) |
|---|---|
| D-glucose monohydrate | 27.8 |
| Sodium chloride | 60.5 |
| Lactic acid | 26.3 |
| Potassium hydroxide | 25 |
| Acetic acid | 16.7 |
| Urea | 6.8 |
| Calcium hydroxide | 3 |
| Glycerol | 1.7 |
| Bovine serum albumin | 0.018 g/L |

**Seminal fluid simulant (SFS)**

| Component | Concentration (mM) |
|---|---|
| Sodium phosphate monobasic monohydrate | 6.72 |
| Sodium phosphate dibasic | 60.44 |
| Sodium citrate dihydrate | 27.64 |
| Potassium chloride | 12.8 |
| Potassium hydroxide | 15.74 |
| D-(-)Fructose | 15.1 |
| D-Glucose monohydrate | 5.15 |
| Lactic acid | 8.15 |
| Urea | 7.49 |
| Bovine serum albumin | 0.76 |
| Calcium chloride | 9.1 |
| Magnesium chloride | 4.53 |
| Zinc chloride | 2.5 |

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008154240 A1
- US 8445465 B2
- US 8445465 B